# EUROPEAN PATENT SPECIFICATION

(11) **EP 2 716 224 B1**
(45) Date of publication and mention of the grant of the patent: **29.03.2017**
(21) Application number: 12792627.7
(22) Date of filing: 18.05.2012
(51) Int. Cl.: A61B 6/00, G03B 42/04, H04N 5/32, G01T 1/24

(54) **RADIATION IMAGING SYSTEM**
STRAHLUNGSBILDGEBUNGSSYSTEM
SYSTÈME DE RADIOGRAPHIE

(30) Priority: 02.06.2011 JP 2011123871
(43) Date of publication of application: 09.04.2014
(73) Proprietor: Konica Minolta, Inc., Tokyo 100-7015 (JP)
(72) Inventor: YONEYAMA, Tsutomu, Hino-shi Tokyo 191-8511 (JP)
(74) Representative: Stanners, David Ralph
(86) International application number: PCT/JP2012/062741
(87) International publication number: WO 2012/165171

(56) References cited:
- EP-A1- 2 148 500
- EP-A2- 2 444 824
- WO-A1-2011/001705
- JP-A- 2008 125 903
- JP-A- 2009 219 538
- JP-A- 2009 261 762
- JP-A- 2010 259 688
- JP-A- 2011 153 876
- JP-A- 2011 172 606
- JP-A- 2011 254 971
- JP-A- 2012 045 044
- US-A1- 2005 161 610

## Description

The present invention relates to a radiation imaging system, and in particular, relates to a radiation imaging system which is mounted on a nursing cart or the like to perform radiation imaging with a portable radiation imaging device.

Various kinds of the so-called direct-type radiation imaging device and the so-called indirect-type radiation imaging device have been developed. The direct-type radiation imaging device generates electric charges using detection elements according to the radiation dose of, for example, emitted X-rays and converts the electric charges into electric signals. The indirect-type radiation imaging device converts emitted radiation into electromagnetic waves of another wavelength such as visible light by using, for example, a scintillator, generates electric charges according to the energy of the converted and emitted electromagnetic waves using photoelectric conversion elements such as photodiodes and then converts the electric charges into electric signals (i.e., image data). In the present invention, the detection elements of the direct-type radiation imaging device and the photoelectric conversion elements of the indirect-type radiation imaging device are collectively called radiation detection elements.

Radiation imaging devices of these types are known as flat panel detectors (FPDs), and each used to be formed integrally with a support (or Bucky device) (for example, see Patent Literature 1). Recently, portable radiation imaging devices made by placing radiation detection elements and other parts in a housing have been developed and put into practical use (for example, see Patent Literatures 2 and 3).

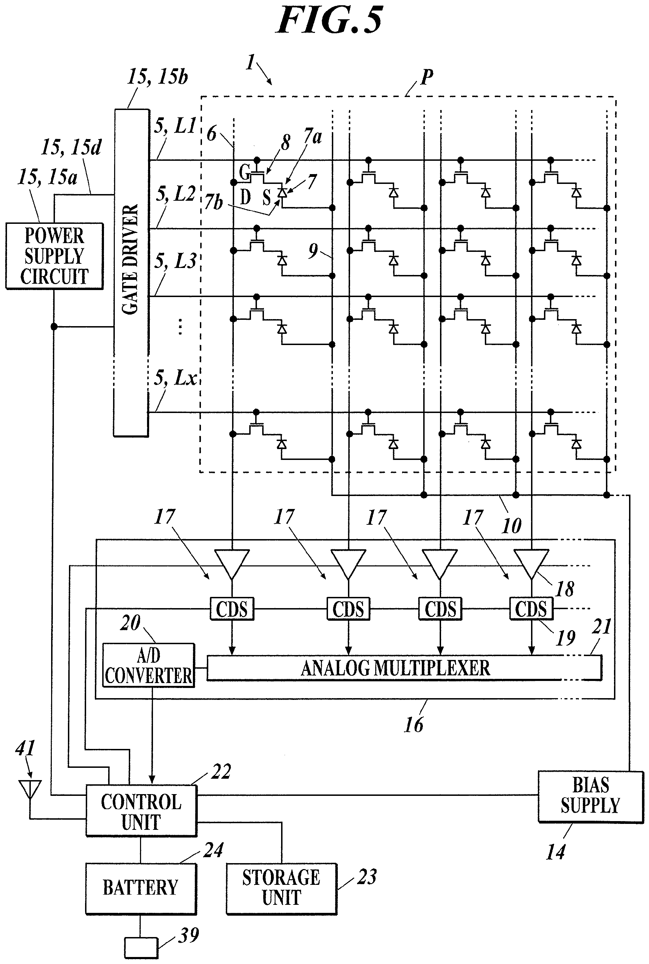

As shown in, for example, FIG. 5 described below, in these radiation imaging devices, normally a plurality of radiation detection elements 7 are arranged two-dimensionally (in a matrix) over a detection unit P, and switch sections constituted of thin film transistors (hereinafter referred to as TFTs) 8 are connected to the respective radiation detection elements 7.

At the time of radiation imaging, OFF voltage is applied to scan lines 5 from a gate driver 15b (see FIG. 5 described later) of a scan driving unit 15, so that the TFTs 8 are in an OFF state. In this state, the radiation imaging device is irradiated from a radiation source through a subject. The irradiation generates electric charges in the radiation detection elements 7 of the radiation imaging device.

After the irradiation, ON voltage is sequentially applied to the scan lines 5 from the gate driver 15b, and the electric charges are read out from the radiation detection elements 7 and undergo electric-charge/voltage conversion in readout circuits 17 to be read out as image data D. In the radiation imaging device, the image data D is thus read out from the radiation detection elements 7.

In order to achieve such radiation imaging, the radiation imaging device disclosed in Patent Literature 4, for example, uses an exposure switch, where a button is operated in two steps, as an exposure switch of a radiation generating device to irradiate a radiation imaging device.

When a radiation technologist performs a first-step operation on the exposure switch, the radiation imaging device is put into a standby state where reverse bias voltage is applied from a bias supply 14 (see FIG. 5 described later) to radiation detection elements 7 for preparation of radiographing. When a second-step operation is performed on the exposure switch, the radiation imaging device is irradiated. After the radiographing, the radiation imaging device reads out image data D from the radiation detection elements 7 as described above.

When the radiation imaging device and the radiation generating device are manufactured by the same manufacturer, radiographing can be performed while signals and information and the like are exchanged between the radiation imaging device and the radiation generating device as described above. The radiation imaging device and the radiation generating device manufactured by different manufacturers, on the other hand, may fail to exchange signals etc. with each other properly.

In such a case, the radiation imaging device applies OFF voltage from the gate driver 15b to the scan lines 5 to put the TFTs 8 in an OFF state after performing a radiation detection element 7 reset process to remove electric charges remaining in the radiation detection elements 7, as described in Patent Literature 3. When being ready to receive radiation in this way, the radiation imaging device may turn on a ready light and notify a radiation technologist that it is ready.

In this case, the radiation technologist operates the exposure switch after the ready light of the radiation imaging device is turned on, for example, to irradiate the radiation imaging device from the radiation generating device.

Patent Literature 1: Japanese Patent Application Laid-Open Publication No. 9-73144
Patent Literature 2: Japanese Patent Application Laid-Open Publication No. 2006-058124
Patent Literature 3: Japanese Patent Application Laid-Open Publication No. 6-342099
Patent Literature 4: Japanese Patent No. 3893181

Unfortunately, the above-described configuration requires a radiographer such as a radiation technologist to wait for a radiographing timing until the ready light is turned on. Further, if a long time is required from the turning on of the ready light to the start of the irradiation of the radiation imaging device, the battery continues to supply electrical power to the functional parts of the radiation imaging device. This easily causes battery drain.

Further, since the TFTs 8 continue to be in the OFF state, so-called dark electric charges accumulate in the radiation detection elements 7 during the OFF state time. The dark electric charges are generated due to thermal excitation, for example, caused by the heat (temperature) of the radiation detection elements 7 themselves. If this state lasts for a long time, more dark electric charges accumulate in the radiation detection elements 7, leading to deterioration in S/N ratio of image data D to be read out.

In order to avoid such problems, irradiation from the radiation generating device may be detected by the radiation imaging device itself and the TFTs 8 may be put into the OFF state at the timing of the detection, such that the electric charges generated in the radiation detection elements 7 due to the irradiation accumulate in the radiation detection elements 7, for example.

The inventor of the present invention et al. have found some effective methods described below as the method of detecting the start of irradiation with the radiation imaging device itself.

These methods read out electric charges leaking via the TFTs 8 as leak data dleak and read out image data d from the radiation detection elements 7 since before the irradiation of the radiation imaging device, as described later. In the following, the image data read out as an actual image after the irradiation is referred to as actual image data D, and the image data read out for irradiation-start detection since before the irradiation is referred to as image data d, as described above.

When the read-out leak data dleak and image data d exceed a predetermined threshold dleak_th and threshold dth, respectively, the start of irradiation is detected.

Unfortunately, with such a configuration, it has been found that the read-out leak data dleak and image data d are sometimes made larger due to some sort of impact on the radiation imaging device. If such an impact increases the leak data dleak to be larger than the threshold dleak_th, for example, there is a danger that the radiation imaging device falsely detects the start of irradiation when irradiation is not executed.

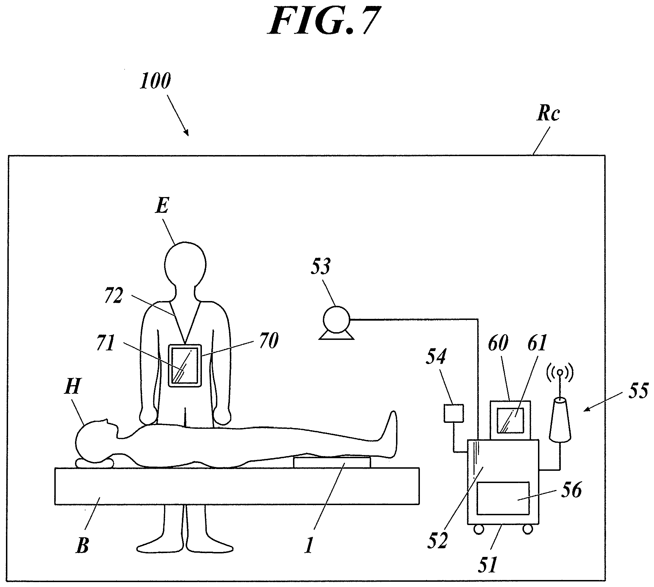

In the case of a radiation imaging device configured to automatically reads out actual image data D and transfers the image data D to a console 60 (see FIG. 7 described later) or subsequently automatically reads out offset data O and transfers the data O upon detecting the start of irradiation, a false detection of the start of irradiation as described above causes automatic readout and transfer of the actual image data D with no subject radiographed. This may waste the electrical power of the battery.

Further, during the actual image data D readout process and the offset data O readout process based on a false detection, the radiation imaging device cannot be used for radiographing. A radiation technologist thus has to operate the radiation imaging device to forcibly stop a series of the above-described processes base on the false detection, for example. This, however, may make the whole radiation imaging system including the radiation imaging device inconvenient.

The present invention has been made in view of the above-described problems and aims to provide a convenient radiation imaging system which can surely stop a series of processes when the radiation imaging device falsely detects the start of irradiation.

The radiation imaging device free from false detection of the start of irradiation does not cause the above-described problems even when subject to an impact.

The present invention also aims to provide a radiation imaging system which can surely prevent the radiation imaging device from falsely detecting the start of irradiation even when the radiation imaging device is subject to an impact.

### MEANS FOR SOLVING PROBLEMS

In order to solve the above-mentioned problems the invention of claim 1 or claim 8 is provided.

In order to solve the above-mentioned problems, a radiation imaging system according to the present disclosure includes: a portable radiation imaging device including: a plurality of scan lines; a plurality of signal lines disposed to intersect with the scan lines; a plurality of radiation detection elements two-dimensionally disposed in small regions formed by the scan lines and the signal lines; a scan driving unit which applies ON voltage or OFF voltage to the scan lines; a plurality of switch sections which are connected to the scan lines and release electric charges accumulated in the radiation detection elements to the signal lines when the ON voltage is applied; a readout circuit which converts the electric charges released from the radiation detection elements to image data and reads out the image data; a control unit which performs a leak data readout process before radiation imaging, the leak data readout process being a process to convert the electric charges leaking from the radiation detection elements via the switch sections to leak data with the scan driving unit applying the OFF voltage to the scan lines so that the switch sections are in an OFF state, the control unit detecting a start of irradiation when the read-out leak data exceeds a threshold; and a communication unit with which the image data is transmitted to an external device; a radiation generating device which controls a radiation source to irradiate the radiation imaging device; a console which generates a preview image and a radiation image on the basis of the image data received from the radiation imaging device; and a portable terminal which transmits a completion signal to the console when the portable terminal receives an input of information indicating that positioning of the radiation imaging device has been completed, wherein, when the control unit of the radiation imaging device detects the start of irradiation, the control unit changes to an electric charge accumulation state and then allows the image data to be read out from the radiation detection elements to transmit part of the read-out image data to the console as data for preview image, the electric charge accumulation state being a state in which the scan driving unit applies the OFF voltage to the scan lines to accumulate electric charges generated due to the irradiation in the radiation detection elements; wherein, when the console receives the data for preview image from the radiation imaging device before receiving the completion signal from the portable terminal, the console transmits a cancel signal to the radiation imaging device, stops a process which is being performed by the radiation imaging device, and makes the leak data readout process before the radiation imaging resume; and wherein, when the console receives the data for preview image from the radiation imaging device after receiving the completion signal from the portable terminal, the console generates the preview image on the basis of the data for preview image.

A radiation imaging system according to the present disclosure includes: a portable radiation imaging device including: a plurality of scan lines; a plurality of signal lines disposed to intersect with the scan lines; a plurality of radiation detection elements two-dimensionally disposed in small regions formed by the scan lines and the signal lines; a scan driving unit which applies ON voltage or OFF voltage to the scan lines; a plurality of switch sections which are connected to the scan lines and release electric charges accumulated in the radiation detection elements to the signal lines when the ON voltage is applied; a readout circuit which converts the electric charges released from the radiation detection elements to image data and reads out the image data; a control unit which performs an image data readout process before radiation imaging, the image data readout process being a process to read out image data for irradiation-start detection from the radiation detection elements with the scan driving unit sequentially applying the ON voltage to the scan lines, the control unit detecting a start of irradiation when the read-out image data exceeds a threshold; and a communication unit with which the image data is transmitted to an external device; a radiation generating device which controls a radiation source to irradiate the radiation imaging device; a console which generates a preview image and a radiation image on the basis of the image data received from the radiation imaging device; and a portable terminal which transmits a completion signal to the console when the portable terminal receives an input of information indicating that positioning of the radiation imaging device has been completed, wherein, when the control unit of the radiation imaging device detects the start of irradiation, the control unit changes to an electric charge accumulation state and then allows the image data to be read out from the radiation detection elements to transmit part of the read-out image data to the console as data for preview image, the electric charge accumulation state being a state in which the scan driving unit applies the OFF voltage to the scan lines to accumulate electric charges generated due to the irradiation in the radiation detection elements; wherein, when the console receives the data for preview image from the radiation imaging device before receiving the completion signal from the portable terminal, the console transmits a cancel signal to the radiation imaging device, stops a process which is being performed by the radiation imaging device, and makes the image data readout process to read out the image data for irradiation-start detection before the radiation imaging resume; and
wherein, when the console receives the data for preview image from the radiation imaging device after receiving the completion signal from the portable terminal, the console generates the preview image on the basis of the data for preview image.

A radiation imaging system according to the present disclosure includes: a portable radiation imaging device including: a plurality of scan lines; a plurality of signal lines disposed to intersect with the scan lines; a plurality of radiation detection elements two-dimensionally disposed in small regions formed by the scan lines and the signal lines; a scan driving unit which applies ON voltage or OFF voltage to the scan lines; a plurality of switch sections which are connected to the scan lines and release electric charges accumulated in the radiation detection elements to the signal lines when the ON voltage is applied; a readout circuit which converts the electric charges released from the radiation detection elements to image data and reads out the image data; a control unit which performs a leak data readout process before radiation imaging, the leak data readout process being a process to convert the electric charges leaking from the radiation detection elements via the switch sections to leak data with the scan driving unit applying the OFF voltage to the scan lines so that the switch sections are in an OFF state, the control unit detecting a start of irradiation when the read-out leak data exceeds a threshold; and a communication unit with which the image data is transmitted to an external device; a radiation generating device which controls a radiation source to irradiate the radiation imaging device; a console which generates a preview image and a radiation image on the basis of the image data received from the radiation imaging device; and a portable terminal which transmits a completion signal to the console when the portable terminal receives an input of information indicating that positioning of the radiation imaging device has been completed, wherein a power consumption mode of the radiation imaging device is switchable between a radiographic mode and a sleep mode, the radiographic mode being a mode in which electric power is supplied from a battery to functional parts including at least the scan driving unit, the readout circuit, and the control unit so that the radiation imaging can be performed, and the sleep mode being a mode in which the radiation imaging is not performed with the electric power supplied only to a necessary functional part including at least the communication unit; wherein the radiation imaging device switches the power consumption mode from the sleep mode to the radiographic mode to start the leak data readout process in response to receipt of a wake-up signal from the console which has received the completion signal from the portable terminal; and wherein, when the control unit of the radiation imaging device detects the start of irradiation, the control unit changes to an electric charge accumulation state and then allows the image data to be read out from the radiation detection elements to transmit part of the read-out image data to the console as data for preview image, the electric charge accumulation state being a state in which the scan driving unit applies the OFF voltage to the scan lines to accumulate electric charges generated due to the irradiation in the radiation detection elements; and wherein the console generates a preview image on the basis of the data for preview image in response to receipt of the data for preview image from the radiation imaging device.

A radiation imaging system according to the present disclosure includes: a portable radiation imaging device including: a plurality of scan lines; a plurality of signal lines disposed to intersect with the scan lines; a plurality of radiation detection elements two-dimensionally disposed in small regions formed by the scan lines and the signal lines; a scan driving unit which applies ON voltage or OFF voltage to the scan lines; a plurality of switch sections which are connected to the scan lines and release electric charges accumulated in the radiation detection elements to the signal lines when the ON voltage is applied; a readout circuit which converts the electric charges released from the radiation detection elements to image data and reads out the image data; a control unit which performs an image data readout process before radiation imaging, the image data readout process being a process to read out image data for irradiation-start detection from the radiation detection elements with the scan driving unit sequentially applying the ON voltage to the scan lines, the control unit detecting a start of irradiation when the read-out image data exceeds a threshold; and a communication unit with which the image data is transmitted to an external device; a radiation generating device which controls a radiation source to irradiate the radiation imaging device; a console which generates a preview image and a radiation image on the basis of the image data received from the radiation imaging device; and a portable terminal which transmits a completion signal to the console when the portable terminal receives an input of information indicating that positioning of the radiation imaging device has been completed, wherein a power consumption mode of the radiation imaging device is switchable between a radiographic mode and a sleep mode, the radiographic mode being a mode in which electric power is supplied from a battery to functional parts including at least the scan driving unit, the readout circuit, and the control unit so that the radiation imaging can be performed, and the sleep mode being a mode in which the radiation imaging is not performed with the electric power supplied only to a necessary functional part including at least the communication unit; wherein the radiation imaging device switches the power consumption mode from the sleep mode to the radiographic mode to start the image data readout process to read out the image data for irradiation-start detection in response to receipt of a wake-up signal from the console which has received the completion signal from the portable terminal;
wherein, when the control unit of the radiation imaging device detects the start of irradiation, the control unit changes to an electric charge accumulation state and then allows the image data to be read out from the radiation detection elements to transmit part of the read-out image data to the console as data for preview image, the electric charge accumulation state being a state in which the scan driving unit applies the OFF voltage to the scan lines to accumulate electric charges generated due to the irradiation in the radiation detection elements; and wherein the console generates a preview image on the basis of the data for preview image in response to receipt of the data for preview image from the radiation imaging device.

According to a radiation imaging system of the present invention, the radiation imaging device 1 performs the readout process to read out leak data dleak and the readout process to read out the image data d for irradiation-start detection before the radiation imaging, and detects the start of irradiation on the basis of the read-out leak data dleak etc. This prevents a time for which the TFTs 8, i.e., the switch sections, are in the OFF state after the detection of the start of irradiation from becoming too long, and surely prevents overconsumption of the power of the battery 24.

Further, the radiation imaging device 1 can surely prevent the problem such as a poor S/N ratio of the read-out image data D caused by increase in dark electric charges accumulating in the radiation detection elements 7 while the TFTs 8 are in the OFF state.

Further, when the console receives data for preview image before receiving the completion signal from the portable terminal i.e., before a radiation technologist completes the positioning of the radiation imaging device, the console can accurately determines that the data for preview image is based on a false detection, and accurately makes the radiation imaging device stop a series of processes. The console can then return the radiation imaging device to the state where the leak data dleak readout process etc. before the radiation imaging is performed.

This eliminates the need for the radiation technologist to wait for the completion of the actual image data readout processes and the completion of the offset data readout process based on a false detection, and enables the radiation technologist to operate the exposure switch immediately for a proper radiographing. The entire radiation imaging system including the radiation imaging device is thus convenient for the radiation technologist.

Further, the power consumption mode of the radiation imaging device may shift from the sleep mode to the radiographic mode upon the console's receipt of the completion signal from the portable terminal when the radiation technologist completes the positioning of the radiation imaging device. This can surely prevent the radiation imaging device, which is in the sleep mode, from falsely detecting the start of irradiation even when the radiation imaging device is subject to an impact while the radiation technologist is performing the positioning of the radiation imaging device.

Further, after the positioning of the radiation imaging device is completed and the completion signal is transmitted from the portable terminal to the console, the power consumption mode of the radiation imaging device shifts to the radiographic mode. This enables the radiation imaging device to accurately detect the start of irradiation, when irradiated, on the basis of the read-out leak data dleak etc.

### BRIEF DESCRIPTION OF DRAWINGS

FIG. 1 is a perspective view showing the external appearance of a radiation imaging device;
FIG. 2 is a sectional view along the line X-X of FIG. 1;
FIG. 3 is a plan view showing the configuration of a board of the radiation imaging device;
FIG. 4 is a side view illustrating the board to which a flexible circuit board and a PCB board and the like are attached;
FIG. 5 is a block diagram showing an equivalent circuit of the radiation imaging device;
FIG. 6 is a block diagram showing an equivalent circuit for one pixel constituting a detection unit;
FIG. 7 illustrates the configuration of a radiation imaging system in accordance with the present embodiment;
FIG. 8 illustrates that electric charges leaking from radiation detection elements via TFTs are read out as leak data;
FIG. 9 is a timing chart showing the timings of turning ON/OFF a switch for resetting electric charges and TFTs in a leak data readout process;
FIG. 10 is a timing chart showing the timings of turning ON/OFF a switch for resetting electric charges, a pulse signal, and TFTs in the case where the leak data readout process and the radiation detection element reset process are alternately performed before the radiation imaging;
FIG. 11 is a timing chart to explain the timings of applying ON voltage to scan lines in a detection method 1;
FIG. 12 is a graph obtained through time-series plotting of the read-out leak data;
FIG. 13 is a graph to explain the way for setting a threshold on the basis of the average and standard deviation of the leak data;
FIG. 14 is a graph to explain the way for setting a threshold on the basis of the average and the difference between the maximum value the minimum value of the leak data;
FIG. 15 is a timing chart showing the timings of sequentially applying ON voltage to the scan lines in the case where an image data readout process to read out image data for irradiation-start detection is repeatedly performed in a detection method 2;
FIG. 16 is a timing chart showing the timings of turning ON/OFF a switch for resetting electric charges, a pulse signal, and TFTs; and an ON time ΔT in the image data readout process to read out image data for irradiation-start detection;
FIG. 17 is a timing chart to explain the timings of applying ON voltage to scan lines in a detection method 2;
FIG. 18 is a timing chart to explain the timings of applying ON voltage to the scan lines in the case where the processing sequence shown in FIG. 11 is repeatedly performed for the offset data readout process;
FIG. 19 is a sequence diagram showing a sequence of communications between and data transmissions to the portable terminal, the console, and the radiation imaging device;
FIG. 20 illustrates a preview image, an "OK" button and an "NG" button displayed on the display screen of the portable terminal;
FIG. 21 illustrates the configuration of a wrong exposure prevention unit and an optical detector provided on the nursing cart of FIG. 7;
FIG. 22A is a side view showing an example of tumbler springs provided at a hinge structure of the wrong exposure prevention unit;
FIG. 22B is an enlarged view of the part of the hinge structure shown from the front;
FIG. 23 is a diagram showing examples of a plurality of radiographic rooms connected to consoles, for example, through a network; and
FIG. 24 illustrates the configuration of an individual radiographic room.

In the following, embodiments of a radiation imaging system in accordance with the present invention are described with reference to the drawings.

In the following, as a radiation imaging device used in the radiation imaging system, a so-called indirect-type radiation imaging device is described. The indirect-type radiation imaging device includes a scintillator and obtains electric signals by converting emitted radiation into electromagnetic waves of another wavelength such as visible light. The present invention can also be applied to a so-called direct-type radiation imaging device which directly detects the radiation with radiation detection elements without using a scintillator or the like.

### [Radiation Imaging Device]

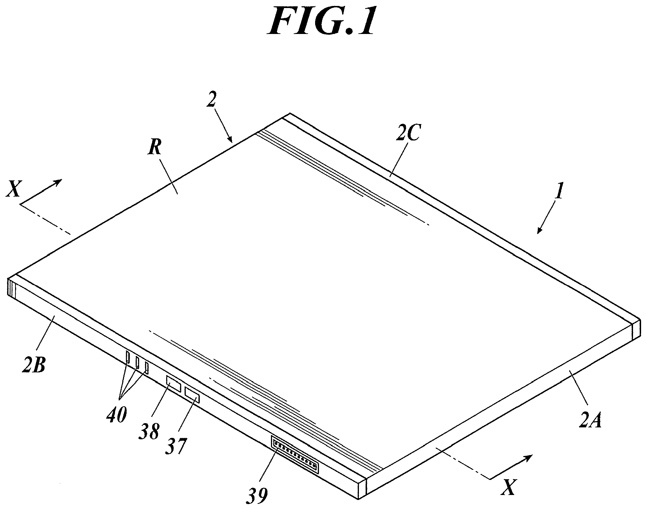

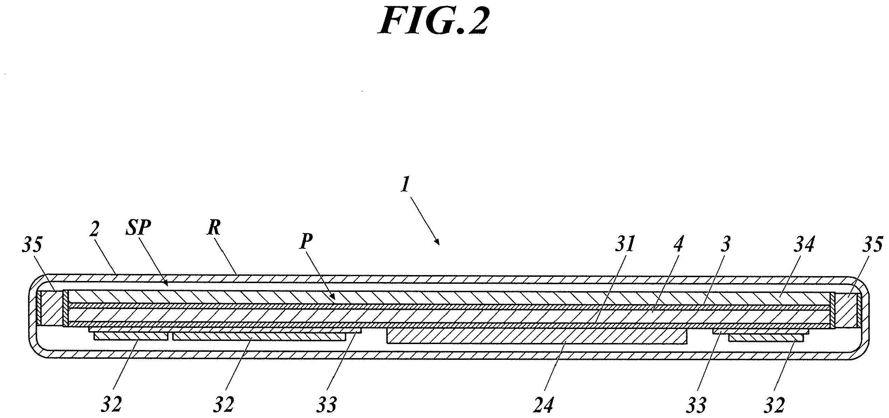

First, the configuration and the like of a radiation imaging device 1 used in a radiation imaging system according to the embodiments are described. FIG. 1 is a perspective view showing the external appearance of the radiation imaging device, and FIG. 2 is a sectional view along the line X-X of FIG. 1.

As shown in FIGS. 1 and 2, the radiation imaging device 1 of the embodiments includes a housing 2 which contains a sensor panel SP constituted of a scintillator 3, a board 4 and the like. In the embodiments, the housing 2 includes a housing main body 2A in the shape of a rectangular hollow cylinder. The housing main body 2A has a radiation incidence plane R and an opening at each side covered with covers 2B and 2C. The radiation imaging device 1 in the embodiments is a portable device having a compatible size with a CR cassette meeting the Japanese Industrial Standards (JIS).

As shown in FIG. 1, one cover 2B of the housing 2 is provided with a power switch 37, a switch 38, a connector 39, and indicators 40 constituted of LEDs, for example, to indicate the state of a battery 24 (see FIGS. 2 and 5 described later) and the operational state of the radiation imaging device 1.

Further, the cover 2C on the other side of the housing 2 is provided with a built-in antenna device 41 (not shown in FIGS 1 and 2; see FIG. 5 described later) to allow the radiation imaging device 1 to wirelessly transmit/receive signals to/from an external device. In the embodiments, the antenna device 41 serves as a communication unit for the radiation imaging device 1 to transmit actual image data D and the like to an external device such as a console 60 described below (see FIG. 7 described below), or for the radiation imaging device 1 to communicate with an external device.

As shown in FIG. 2, the housing 2 contains a base 31 disposed under the board 4 with a not-shown lead sheet placed between the base 31 and the board 4. On the base 31, PCB boards 33 each provided with an electronic component 32, the battery 24 and the like are provided. The incident surfaces R of the board 4 and the scintillator 3 are provided with a glass board 34 to protect them, and cushions 35 are disposed between the sensor panel SP and the lateral surfaces of the housing 2.

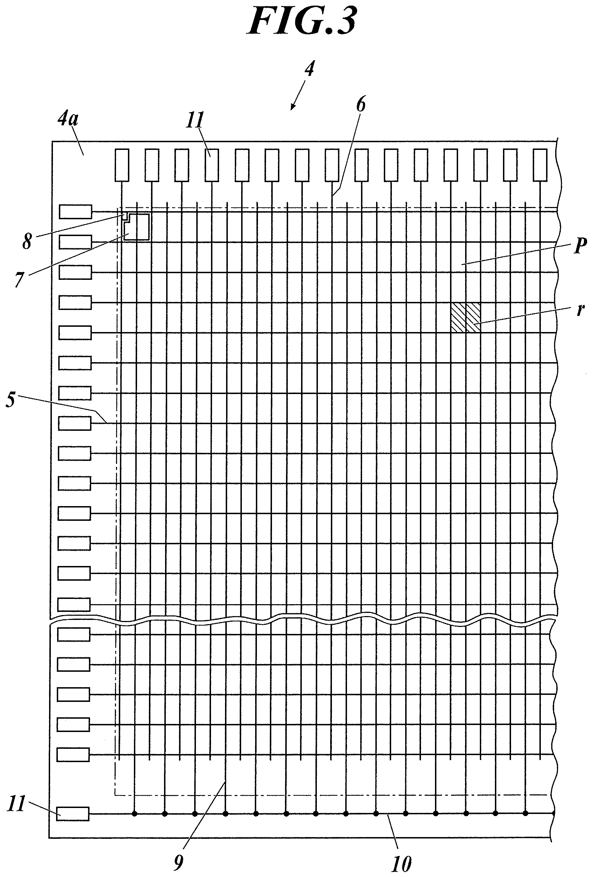

As shown in FIG. 3, a plurality of scan lines 5 and signal lines 6 are arranged so as to intersect each other on the detection unit P of the board 4. Radiation detection elements 7 constituted of photodiodes or the like are two-dimensionally arranged in matrix form in the respective small regions r formed by the scan lines 5 and the signal lines 6.

The radiation detection elements 7 are connected to respective thin film transistors (hereinafter referred to as TFTs) 8 as switch sections, and are further connected to bias lines 9. The bias lines 9 are connected to a connection line 10 outside of the detection unit P of the board 4.

In the embodiments, as shown in FIG. 3, the scan lines 5, the signal lines 6, and the connection line 10 to which the bias lines 9 are connected are connected to input/output terminals (also referred to as pads) 11 which are disposed near the edges of the board 4.

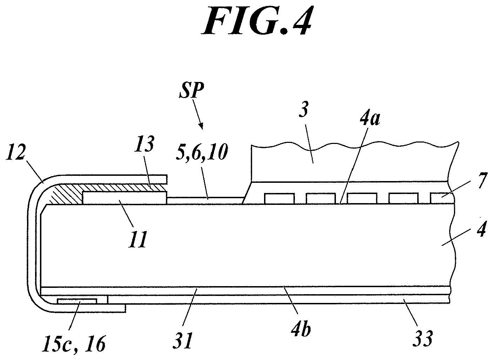

As shown in FIG. 4, a flexible circuit board (also referred to as Chip On Film) 12 is connected to each input/output terminal 11 through an anisotropic conductive adhesive material 13 such as an anisotropic conductive film or an anisotropic conductive paste. The flexible circuit board 12 includes chips, such as a gate IC 15c constituting the gate driver 15b of the scan driving unit 15, described later, and a readout IC 16, embedded in its film.

The flexible circuit board 12 extends to the back surface 4b of the board 4, and connected to the above-described PCB board 33 on the back surface 4b. The scintillator 3 is disposed so as to face the detection unit P of the board 4. Thus, the sensor panel SP of the radiation imaging device 1 is formed. In FIG. 4, the electronic components 32 and the like are not shown.

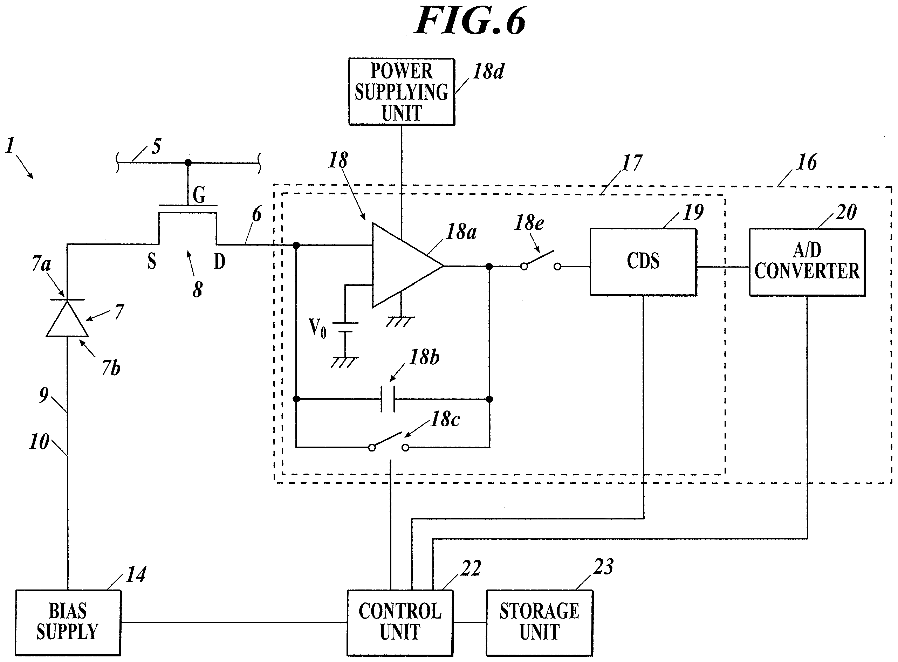

Here, the circuit configuration of the radiation imaging device 1 is described. FIG. 5 is a block diagram showing an equivalent circuit of the radiation imaging device 1 of the embodiments. FIG. 6 is a block diagram showing an equivalent circuit for one pixel constituting the detection unit P.

Each radiation detection elements 7 of the detection unit P on the board 4 has a second electrode 7b connected to a bias line 9, and each bias line 9 is connected to the connection line 10 so as to be connected to a bias power supply 14. The bias power supply 14 applies bias voltage to the second electrodes 7b of the radiation detection elements 7 via the connection line 10 and the bias lines 9. The bias supply 14 is connected to a control unit 22, described later, which controls the bias voltage applied from the bias supply 14 to the radiation detection elements 7.

In the embodiments, as shown in FIGS. 5 and 6, the bias power supply 14 applies a voltage (i.e., so-called reverse bias voltage) to the second electrodes 7b of the radiation detection elements 7 via the bias lines 9 as the bias voltage, the voltage being equal to or less than a voltage applied to the first electrodes 7 a of the radiation detection elements 7.

The scan driving unit 15 includes a power supply circuit 15a and the gate driver 15b. The power supply circuit 15a supplies ON voltage and OFF voltage to the gate driver 15b via a line 15d. The gate driver 15b switches the voltage to be applied to lines L1-Lx of the scan lines 5 between ON voltage and OFF voltage to switch the state of the TFTs 8 between an ON state and OFF state.

The signal lines 6 are connected to readout circuits 17 embedded in the readout IC 16. Each of the readout circuits 17 is constituted of an amplifier circuit 18, a correlated double sampling circuit 19 and the like. In the readout IC 16, an analog multiplexer 21 and an A/D converter 20 are further provided. In FIGS. 5 and 6, each correlated double sampling circuit 19 is represented by "CDS". The analog multiplexer 21 is omitted in FIG. 6.

In the embodiments, the amplifier circuits 18 are each constituted of a charge amplifier circuit including a power supplying unit 18d. The power supplying unit 18d is connected to an operational amplifier 18a, a capacitor 18b, and a switch 18c for resetting electric charges; and supplies electrical power to the operational amplifier 18a etc. The capacitor 18b and the switch 18c are connected in parallel to the operational amplifier 18a. A signal line 6 is connected to the inverting input terminal of the input side of the operational amplifier 18a of the amplifier circuit 18. A reference potential V₀ is applied to the non-inverting input terminal of the input side of the amplifier circuit 18. The reference potential V₀ is set to an appropriate value, and in the embodiments, 0[V] is applied.

The switch 18c for resetting electric charges of each amplifier circuit 18 is connected to the control unit 22 which performs ON/OFF control. A switch 18e is disposed between the operational amplifier 18a and the correlated double sampling circuit 19. The switch 18e opens/closes in conjunction with the switch 18c for resetting electric charges. The switch 18e is turned OFF/ON in conjunction with the ON/OFF operation of the switch 18c for resetting electric charges.

At the time of a radiation detection element 7 reset process for removing electric charges remaining in the radiation detection elements 7 of the radiation imaging device 1, the TFTs 8 are in the ON state while the switch 18c for resetting electric charges is in the ON state (and the stich 18e is in the OFF state).

The radiation detection elements 7 then release electric charges via the TFTs 8 to a signal line 6. The electric charges then pass through the switch 18c for resetting electric charges of the amplifier circuit 18, go into the operational amplifier 18a from its output terminal, and go out from the non-inverting input terminal to be earthed or flow out to the power supplying unit 18d.

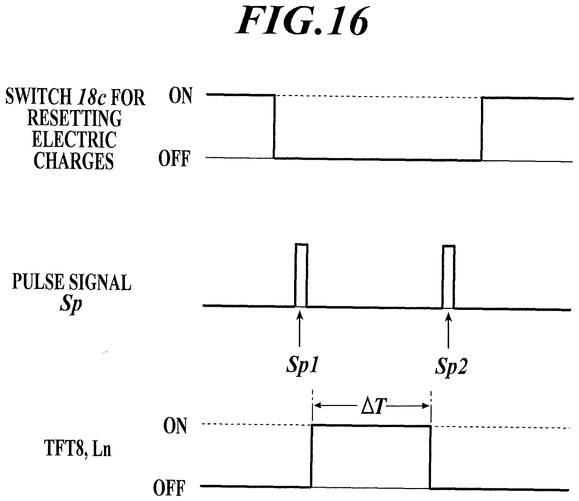

As shown in FIG. 16, described later, at the time of a readout process to read out the image data D as an actual image from the radiation detection elements 7 and a later-described readout process to read out the image data d for irradiation-start detection, electric charges are released from the radiation detection elements 7 to a signal line 6 via the ON-state TFTs 8, while the switch 18c for resetting electric charges of the amplifier circuit 18 is in the OFF state (and while the switch 18e is in the ON state). The electric charges then accumulate in the capacitor 18b of the amplifier circuit 18.

The voltage value according to the amount of electric charges accumulated in the capacitor 18b of an amplifier circuit 18 is output from the output side of the operational amplifier 18a. When a pulse signal Sp1 is transmitted from the control unit 22 before the electric charges flow out from the radiation detection elements 7, the correlated double sampling circuit (CDS) 19 holds the voltage value Vin outputted from the amplifier circuit 18 at the time.

When a pulse signal Sp2 is transmitted from the control unit 22 after the electric charges flowing out from the radiation detection elements 7 accumulate in the capacitor 18b of the amplifier circuit 18, voltage value Vfi outputted from the amplifier circuit 18 at the time is held. The difference (Vfi - Vin) between the voltage values is calculated to be output to the downstream side as image data D of an analog value.

The image data D of the radiation detection elements 7 outputted from the correlated double sampling circuit 19 is sequentially transmitted to the A/D converter 20 via the analog multiplexer 21. The image data D is then converted into digital image data sequentially by the A/D converter 20, outputted to the storage unit 23, and sequentially stored therein.

After the completion of one image data readout process, the switch 18c for resetting electric charges of the amplifier circuit 18 is turned on and the electric charges accumulated in the capacitor 18b are released. The released electric charges then go into the operational amplifier 18a from its output terminal and go out from the non-inverting input terminal to be earthed or flow out to the power supplying unit 18d, as in the same manner described above, for resetting the amplifier circuit 18.

The control unit 22 is constituted of a microcomputer in which a not-shown central processing unit (CPU), a read only memory (ROM), a random access memory (RAM), an input/output interface, and the like are connected by a bus; a field programmable gate array (FPGA); or the like. The control unit 22 controls the operations of the components of the radiation imaging device 1.

The control unit 22 is connected to the storage unit 23. The storage unit 23 is constituted of a static RAM (SRAM), a synchronous DRAM (SDRAM) or the like. In the embodiments, the control unit 22 is connected to the above-described antenna device 41, the power source switch 37, the switch 38, the connector 39, the indicators 40 (see FIG. 1), and the like.

Further, the control unit 22 is connected to the battery 24 to supply power to the functional parts such as the control unit 22, the scan driving unit 15, the readout circuits 17, the storage unit 23, and the bias supply 14. The above-described connector 39 is connected to the battery 24 to charge the battery through the connector 39.

As described above, the inventor of the present invention et al. have found new methods for detecting the start of irradiation with the radiation imaging device 1 itself having such a configuration. These methods are described after the description of the radiation imaging system 100.

### [Radiation Imaging System]

Next, the configuration and the like of the radiation imaging system 100 for radiographing using the radiation imaging device 1 according to the embodiments are described. FIG. 7 illustrates the configuration of the radiation imaging system in accordance with the embodiments. In FIG. 7, the radiation imaging system 100 is constructed on a nursing cart 51 so that the entire system is portable.

As shown in FIG. 7, in the radiation imaging system 100 in accordance with the embodiments, the radiation imaging device 1 is used by being inserted between a bed B installed in a hospital room Rc, for example, and the body of a patient B lying on the bed B; or directly placed on the body of the patient. Hence, the housing 2 is easily subject to an impact at the time of positioning of the radiation imaging device 1. In the case of the housing 2 having a compatible size with the CR cassette as described above, in particular, the space in the housing 2 is small, which constrains the locations of the cushions. A heavy impact, therefore, may influence a generated captured image.

A radiation generating device 52 is mounted on the nursing cart 51. The radiation generating device 52 is connected with a portable radiation source 53 to irradiate the radiation imaging device 1 through the body of the patient B, i.e., a subject. Further, an exposure switch 54 to be operated by a radiation technologist E to instruct the radiation source 53 to start irradiation is attached to the radiation generating device 52. The exposure switch 54 may be of a type where a not-shown button is operated in two steps, which is described above as a conventional exposure switch, for example.

An access point (also referred to as a radio antenna etc.) 55 for wireless communications with the antenna device 41 of the radiation imaging device 1 (see FIG. 5) is attached to the radiation generating device 52. The access point 55 is connected with a not-shown relay provided inside the radiation generating device 52.

The relay relays communications through a local area network (LAN) between the radiation imaging device 1 and the later-described portable terminal 70 and console 60. The relay converts signals for LAN communications into signals for the radiation generating device 52, for example, and vice versa to relay communications between the console 60 and the radiation generating device 52.

In the embodiments, a holder 56 is provided on a lateral face of the radiation generating device 52. The radiation imaging device 1 is inserted in the holder 56 to be carried with the nursing cart 51.

The holder 56 may be formed simply as a pocket, as it were, to hold the radiation imaging device 1. Additionally, in the embodiments, inserting the radiation imaging device 1 into the holder 56 further allows the connector 39 (see FIG. 1) of the radiation imaging device 1 to be connected with a not-shown connector provided in the holder 56 so as to automatically charge the battery 24 (see FIGS. 2 and 5) of the radiation imaging device 1.

In the embodiments, a power consumption mode of the radiation imaging device 1 is switchable between a radiographic mode (also referred to as a wake up mode etc.) and a sleep mode (also referred to as a power saving mode etc.) in terms of a power consumption state of the battery 24. The radiographic mode is a mode in which electrical power is supplied to the functional parts such as the scan driving unit 15 and the readout circuits 17 (see FIG. 5) so that radiation imaging can be performed. The sleep mode is a mode in which electrical power is not supplied to the scan driving unit 15 and the readout circuits 17 etc. but is supplied only to a necessary functional part such as the antenna device 41.

As described above, the power consumption mode of the radiation imaging device 1 is the sleep mode while the device 1 is placed in the holder 56 to be charged. On the other hand, the radiation imaging device 1 automatically shifts to the radiographic mode in response to the disconnection of the connector 39 of the radiation imaging device 1 from the connector in the holder 56 when the radiation imaging device 1 is pulled out of the holder 56.

In the embodiments, the console 60 is placed on the radiation generating device 52 of the nursing cart 51. As described above, the console 60 is connected with the radiation generating device 52 and the access point 55 via the relay to communicate with the radiation imaging device 1 and the later-described portable terminal 70 via the access point 55.

In the embodiments, the console 60 is constituted of a computer including a not-shown CPU and the like. The console 60 is provided with a display unit 61 including a cathode ray tube (CRT), a liquid crystal display (LCD), and the like. Further, a storage unit 59 constituted of a hard disk drive (HDD) or the like is connected to or embedded in the console 60.

The console 60 is basically configured to generate a preview image on the basis of data for preview image received from the radiation imaging device 1 to display the generated preview image on the display unit 61, as described later.

The console 60 performs predetermined image processing, such as offset correction, gain correction, defective pixel correction, and gradation processing, on actual image data D and offset data O received from the radiation imaging device 1 to generate a radiation image for diagnosis in the embodiments, as described later.

In the embodiments, when the radiation imaging device 1 falsely detects the start of irradiation, reads out actual image data D, and transmits the data for preview image based on the actual image data D to the console 60, the console 60 does not accept the data, as described later. This process is described later in detail after the description of new methods for detecting the start of irradiation with the radiation imaging device 1 itself found by the inventor of the present invention et al.

In the embodiments, the radiation imaging system 100 includes the portable terminal 70 as shown in FIG. 7. The portable terminal 70 wirelessly transmits a completion signal to the console 60 when a radiation technologist E inputs the information indicating the completion of positioning of the radiation imaging device 1 at the completion of the positioning of the radiation imaging device 1 inserted between a bed B and the body of a patient B or directly placed on the body of the patient.

The portable terminal 70 is basically carried by the radiation technologist E. As shown in FIG. 7, the portable terminal 70 is preferably dangled around the neck of the radiation technologist E with a strap 72, for example, not to interfere with the positioning of the radiation imaging device 1. A portable information terminal which allows input operation, such as an iPad (registered trademark), may be used as the portable terminal 70.

The portable terminal 70 preferably includes a display screen 71 as shown in FIG. 7, which is described later. The portable terminal 70 does not necessarily need to be a general-purpose information terminal that is commercially available, such as an iPad (registered trademark), but may be a portable information terminal for exclusive use which allows input operation. Alternatively, the portable terminal 70 may be something like a simple switch including an antenna device etc.

### [Configuration of Detection of Irradiation Start]

Next, detecting processes for achieving new methods to detect the start of irradiation with the radiation imaging device 1 in accordance with the embodiments, which have been found by the inventor of the present invention et al., are described. Any one of the following two detection methods may be used, for example.

### [Detection Method 1]

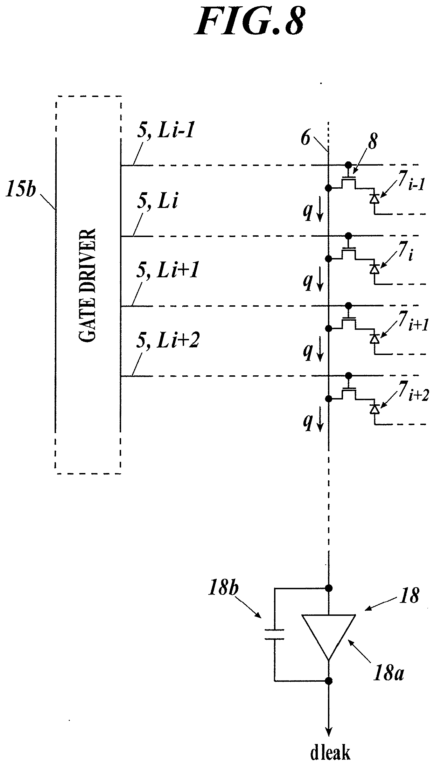

The radiation imaging device 1 can be configured to repeatedly perform a leak data dleak readout process before being irradiated in radiation imaging, for example. The leak data dleak is the data equivalent to the sum of values of the electric charges q for each signal line 6 leaking from the radiation detection elements 7 via the OFF-state TFTs 8 in a state in which OFF voltage is applied to the scan lines 5, as shown in FIG. 8.

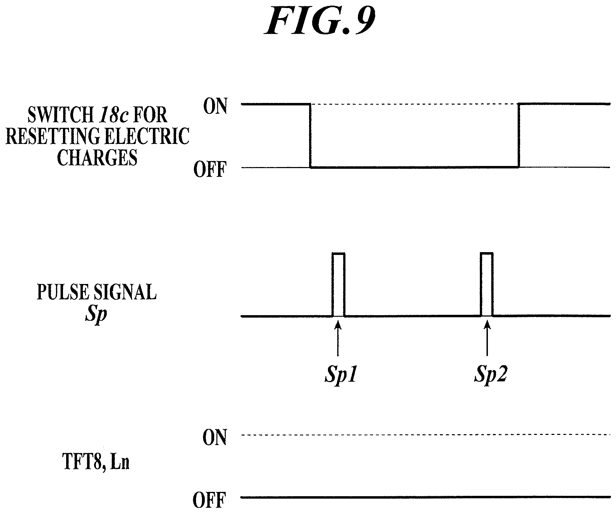

Unlike a image data d readout process shown in FIG. 16, described later, and an actual image data D readout process to be performed in the same manner as the image data d readout process, the leak data dleak readout process makes a readout circuit 17 perform a readout process in a state in which the OFF voltage is applied to lines L1-Lx of the scan lines 5 to put the TFTs 8 in the OFF state, as shown in FIG. 9.

Specifically, as shown in FIG. 9, the control unit 22 transmits pulse signals Sp1 and Sp2 to the correlated double sampling circuit 19 (see CDS of FIG. 5) of a readout circuit 17 in a state in which the OFF voltage is applied to lines L1-Lx of the scan lines 5 to put the TFTs 8 in the OFF state. In response to receipt of the pulse signal Sp1 from the control unit 22, the correlated double sampling circuit 19 holds the voltage value Vin outputted from the amplifier circuit 18 at this time.

When electric charges q leaking from the radiation detection elements 7 via the TFTs 8 are accumulated in the capacitor 18b of the amplifier circuit 18, the voltage value outputted from the amplifier circuit 18 increases and the control unit 22 transmits the pulse signal Sp2. The correlated double sampling circuit 19 then holds the voltage value Vfi outputted from the amplifier circuit 18 at this time.

The correlated double sampling circuit 19 outputs the value obtained by calculating the difference (Vfi - Vin) between the voltage values. This outputted value is the leak data dleak. The leak data dleak is then converted into a digital value by the A/D converter 20 as in the case of the above-described image data D readout process. The leak data dleak readout process is thus performed.

Repeatedly performing only the leak data dleak readout process, however, maintains the TFTs 8 in the OFF state, causing the dark electric charges generated in the radiation detection elements 7 to continue to accumulate in the radiation detection elements 7.

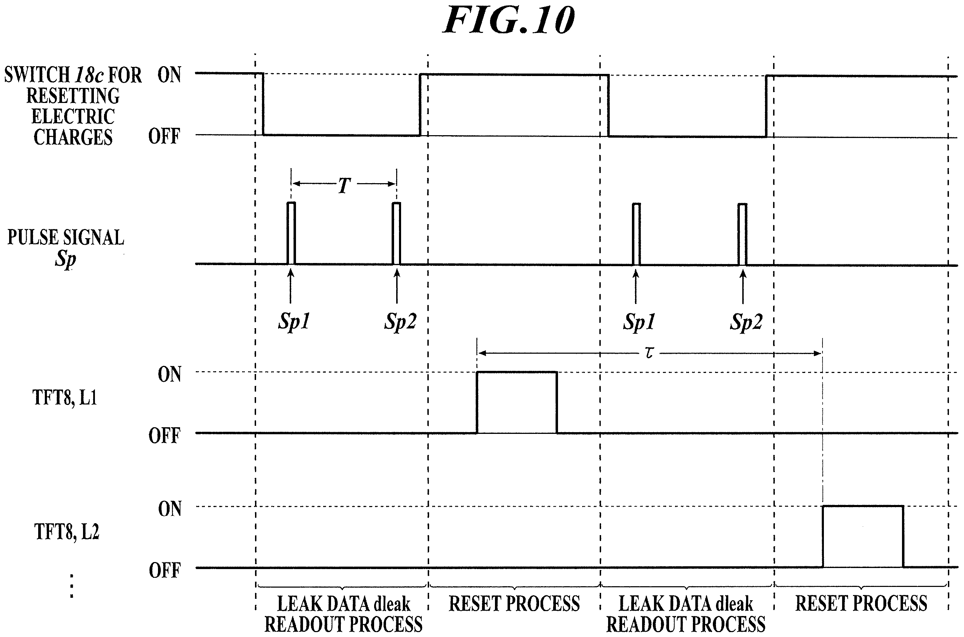

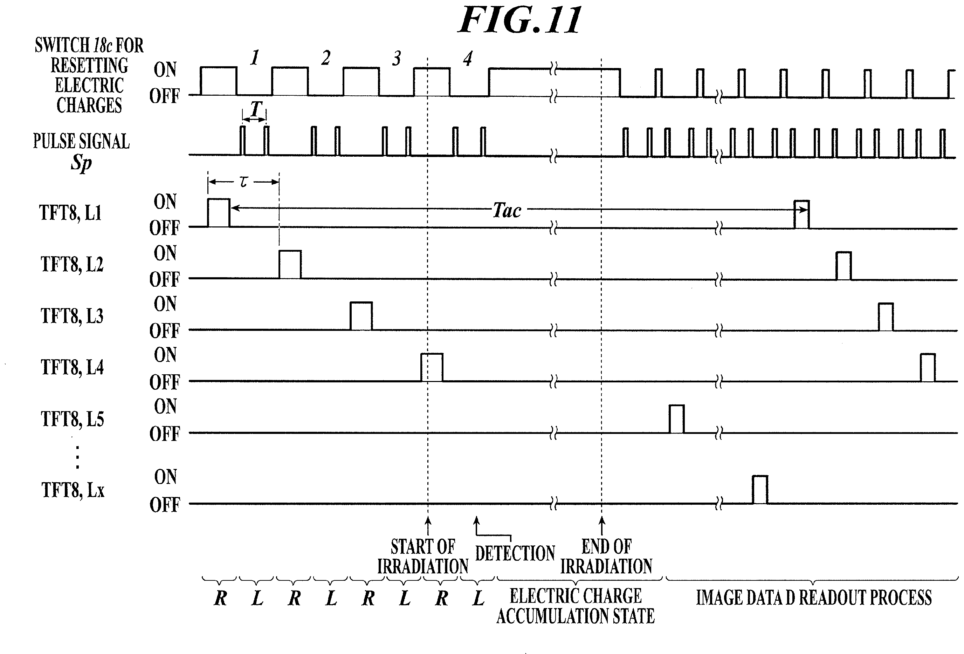

In the detection method 1, therefore, the leak data dleak readout process, which is performed with the OFF voltage applied to the scan lines 5, and the radiation detection element 7 reset process, where the ON voltage is sequentially applied to lines L1-Lx of the scan lines 5, are preferably performed alternately as shown in FIG. 10. Explanations for "T" and "τ" in FIGS. 10 and 11 are described later.

In the case where the leak data dleak readout process and the radiation detection element 7 reset process are alternately performed before radiation imaging as described above, electromagnetic waves into which the radiation has been converted with the scintillator 3 (see FIG. 2) are put onto the TFTs 8 upon the start of irradiation of the radiation imaging device 1 from the radiation source 53 (see FIG. 7).

The studies conducted by the inventor of the present invention et al. have found out that such exposure of the TFTs 8 to the electromagnetic waves increases the electric charges q leaking from the radiation detection elements 7 via the TFTs 8 (see FIG. 8).

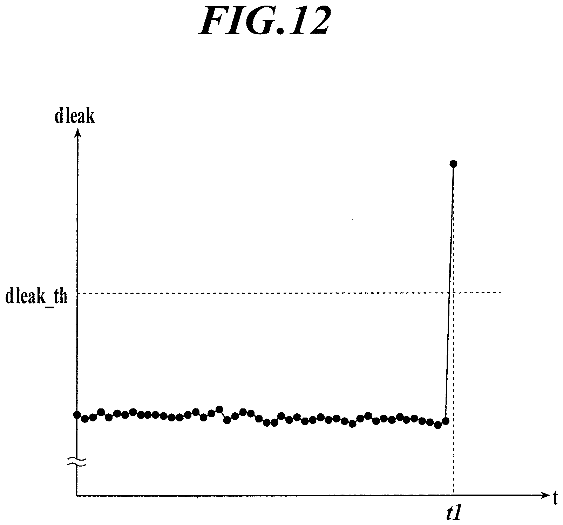

In the case where the leak data dleak readout process and the radiation detection element 7 reset process are alternately performed before the radiation imaging as shown in FIG. 11, the leak data dleak read out at the timing of the start of irradiation of the radiation imaging device 1 is much larger than the leak data dleak which was read out before the irradiation start, as shown in FIG. 12.

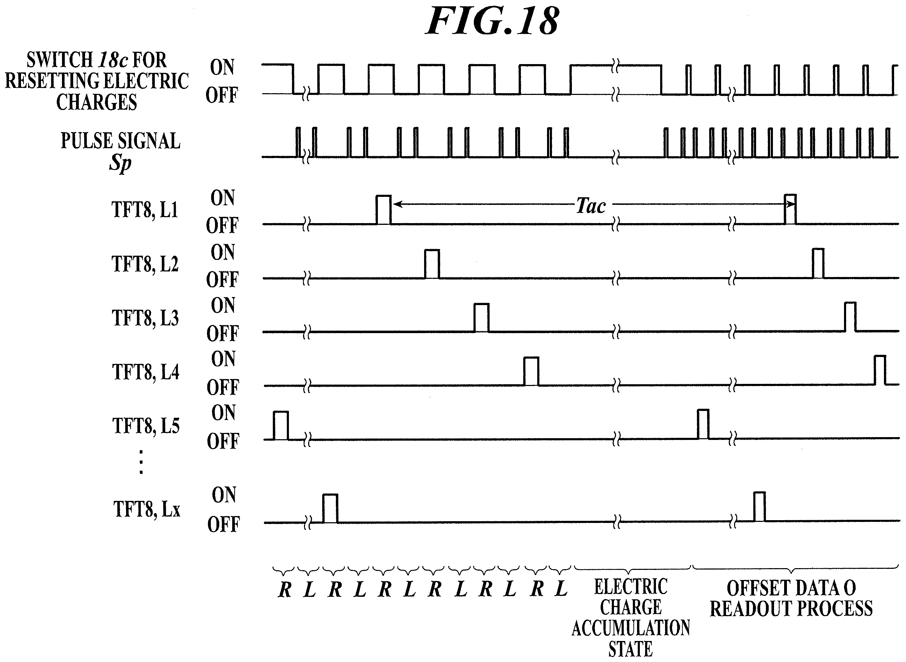

As for FIGS. 11 and 12, the leak data dleak read out in the fourth readout process after the ON voltage is applied to line L4 of the scan lines 5 and the reset process is performed in FIG. 11 corresponds to the leak data dleak at the time t1 in FIG. 12. In FIG. 11 and FIG. 18, described later, "R" represents the radiation detection element 7 reset process, and "L" represents the leak data dleak readout process. The sign "Tac" in FIG. 11 is described later.

The control unit 22 of the radiation imaging device 1 may be configured to monitor the leak data dleak read out in the leak data dleak readout process before the radiation imaging, and configured to detect the start of irradiation at the timing when the read-out leak data dleak exceeds a preset predetermined threshold dleak_th (see FIG. 12), for example.

Here, the method for setting the threshold dleak_th in the embodiments is described. The value of leak data dleak read out without the irradiation of radiation imaging device 1 (i.e., the value of leak data dleak before the time t1) is almost constant, but fluctuates a little as shown in FIG. 12. When such fluctuating leak data dleak exceeds the threshold dleak_th, the start of irradiation is falsely detected although irradiation has not actually occurred. The threshold dleak_th, therefore, needs to be set to such a value that the fluctuating leak data dleak does not exceed the value.

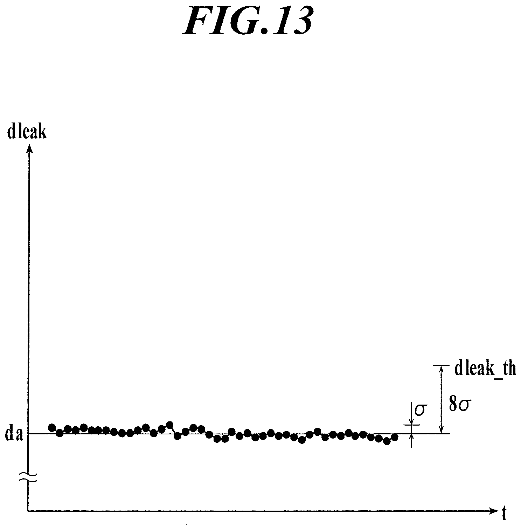

In view of this, in the embodiments, the average da of the leak data dleak read out in predetermined-times readout processes is calculated when the value of leak data dleak read out without irradiation of the radiation imaging device 1 becomes stable as shown in FIG. 13. Further, a standard deviation σ is calculated, for example, as the degree of fluctuation of the leak data dleak.

The value (8σ, for example) obtained by multiplying the standard deviation σ by a predetermined number, such as 8, is added to the average da of the leak data dleak to obtain the value da+ 8σ, which is set as the threshold dleak_th.

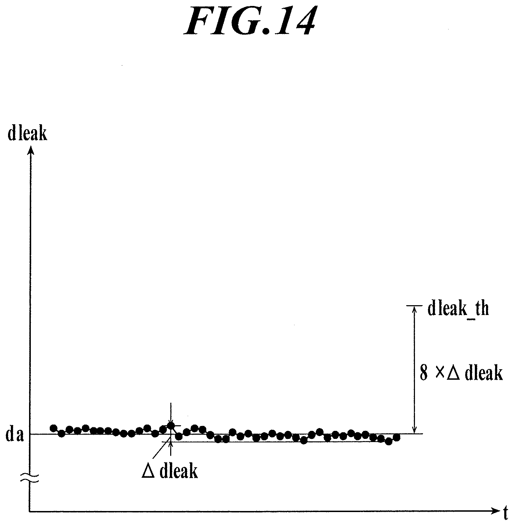

Instead of using the standard deviation σ and the dispersion σ2 of the leak data dleak as the degree of fluctuation of the leak data dleak as described above, the difference Δdleak between the maximum and minimum values of the leak data dleak may be used as shown in FIG. 14. In this case, the threshold dleak_th is set by calculating da + 8×Δdleak, that is, adding the value (8×Δdleak, for example) obtained by multiplying the difference Δdleak between the maximum and minimum values of the leak data dleak by a predetermined number, such as 8, to the average da of the leak data dleak, as shown in FIG. 14.

In the embodiments, the threshold dleak_th for the leak data dleak is preset as described above. In the above-described case, a number by which the standard deviation σ or the difference Δdleak is multiplied is determined as appropriate. A threshold dth for image data d in the detection method 2, described below, is similarly set.

### [Detection Method 2]

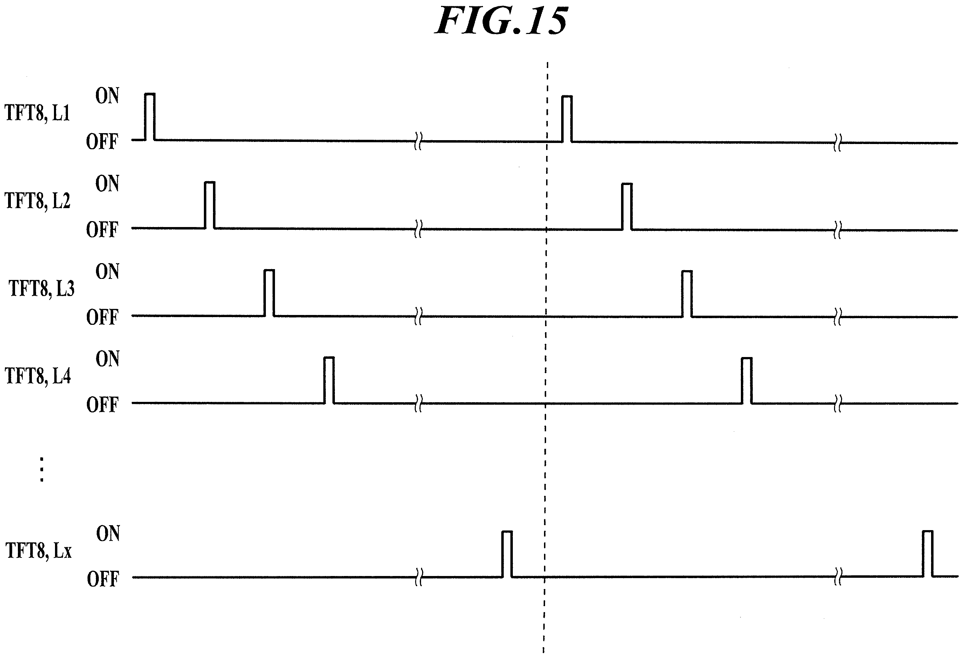

Instead of performing the leak data dleak readout process before the radiation imaging as in the detection method 1, an image data d readout process to read out image data d for irradiation-start detection from the radiation detection elements 7 may be performed by sequentially applying ON voltage to lines L1-Lx of the scan lines 5 from the gate driver 15b of the scan driving unit 15 before the radiation imaging as shown in FIG. 15.

In this case, the switch 18c for resetting electric charges of the amplifier circuit 18 of the readout circuit 17 is turned ON and OFF and the pulse signals Sp1 and Sp2 are transmitted to the correlated double sampling circuits 19 in the image data d readout process in the same manner as in the actual image data D readout process as shown in FIG. 16. The sign "ΔT" in FIG. 16 etc. is described later.

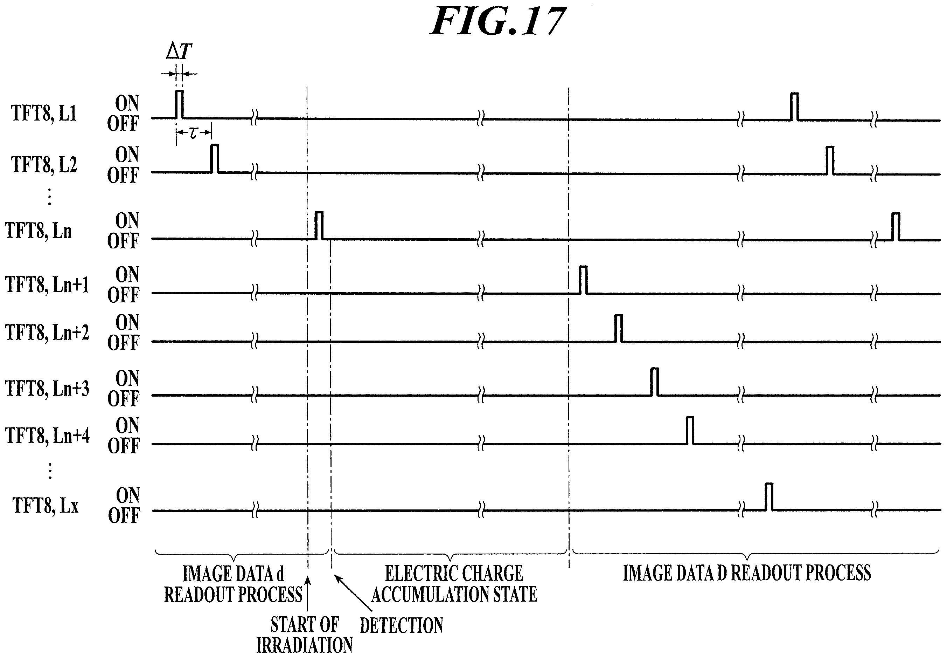

In the case where the image data d readout process is performed before the radiation imaging as described above, as shown in FIG. 17, the image data d read out at the timing of the start of irradiation of the radiation imaging device 1 (i.e., the image data d read out in response to the application of the ON voltage to the line Ln of the scan lines 5 in FIG. 17) is much larger than the image data d read out before the irradiation start, similarly to the case of the leak data dleak shown in FIG. 12.

In view of this, the control unit 22 of the radiation imaging device 1 may be configured to monitor the image data d read out in the readout process before the radiation imaging, and configured to detect the start of irradiation at the timing when the read-out image data d exceeds a preset predetermined threshold dth.

In order to improve the sensitivity to read out the leak data dleak and image data d in the detection methods 1 and 2, the cycle τ of the leak data dleak readout process and the image data d readout process (see FIGS. 10, 11, and 17), the transmission interval T between the pulse signals Sp1 and Sp2 (see FIGS. 10 and 11), and the time ΔT for which the ON voltage is applied to the TFTs 8 may be longer.

### [Process after Detection of Irradiation Start]

Next, the processes to be performed by the control unit 22 of the radiation imaging device 1 after the detection of the start of irradiation as described above are described. In the following, the case is described where the detection method 1 is used as the method of detecting the start of irradiation. The processes are similarly performed when the detection method 2 is employed.

Upon detecting the start of irradiation as described above, the control unit 22 stops applying the ON voltage to the scan lines 5, applies the OFF voltage to lines L1-Lx of the scan lines 5 from the gate driver 15b to put the TFTs 8 into the OFF state, and shifts to an electric charge accumulation state where the electric charges generated in the radiation detection elements 7 through the irradiation are accumulated in the radiation detection elements 7, as shown in FIG. 11.

When a predetermined time has passed since the detection of irradiation start, the irradiation ends. After that, the control unit 22 starts applying the ON voltage from the scan line 5 to which the ON voltage is to be applied (i.e., the line L5 of the scan lines 5 in the case of FIG. 16) next to the scan line 5 to which the ON voltage was applied (i.e., the line L4 of the scan lines 5 in the case of FIG. 16) in the reset process immediately before the detection of irradiation start in the leak data dleak readout process before the radiation imaging, for example. The control unit 22 then sequentially applies the ON voltage to the scan lines 5 to perform an actual image data D readout process to read out image data D as an actual image.

Alternatively, the application of the ON voltage may be started with the first line L1 of the scan lines 5, so that the ON voltage is sequentially applied to the scan lines 5 to perform the actual image data D readout process.

In the embodiments, when the radiation imaging is performed and the actual image data D is read out from the radiation detection elements 7, the control unit 22 first losslessly compresses the actual image data D read out from predetermined part, such as 1/4, of all of the radiation detection elements 7, e.g., the radiation detection elements 7 connected to the lines L1, L5, L9, ... of the scan lines 5. The control unit 22 then automatically transmits the losslessly-compressed image data D to the console 60 (see FIG. 7) through the antenna device 41 (see FIG. 5) as the data for preview image.

In the embodiments, the control unit 22 repeats the processing sequence, which is the same processing sequence as the one performed before the actual image data D readout process shown in FIG. 11 to read out offset data O from the radiation detection elements 7 as shown in FIG. 18 after the actual image data D readout process and the transmission of the data for preview image.

The offset data O readout process is performed without irradiating the radiation imaging device 1. The data equivalent to the offset caused by the so-called dark electric charges superposed on the image data D read out from the radiation detection elements 7 as described above is read out as offset data O.

When completing the offset data O readout process described above, the control unit 22 compresses and transmits the offset data O read out from the predetermined part, such as 1/4, of the radiation detection elements 7, and compresses the image data D and the offset data O for the remaining 3/4 of the radiation detection elements 7 to transmit the compressed image data D and the offset data O to the console 60.

Performing the offset data O readout process by repeating the same processing sequence as the processing sequence performed before the actual image data D readout process described above allows the time Tac (hereinafter referred to as effective accumulation time) in FIG. 11 and the effective accumulation time Tac in FIG. 18 to be the same for each scan line 5. The time Tac in FIG. 11 is the effective accumulation time from when the ON voltage is applied to the TFTs 8 immediately before the actual image data D readout process to when the ON voltage is applied in the actual image data D readout process. The time Tac in FIG. 18 is the effective accumulation time in the offset data O readout process.

The amount of the dark electric charges read out from the radiation detection elements 7 changes depending on the time for which the TFTs 8 connected to the radiation detection element 7 are in the OFF state, i.e., the effective accumulation time Tac in FIGS. 11 and 18. The same effective accumulation time Tac thus results in the same amount of the dark electric charges read out from the radiation detection elements 7.

In view of this, performing the offset data O readout process by repeating the same processing sequence as the processing sequence performed before the actual image data D readout process as described above allows the effective accumulation time Tac for each scan line 5 to be the same between the actual image data D readout process (see FIG. 11) and the offset data O readout process (see FIG. 18).

Accordingly, the amount of the dark electric charges read out from the radiation detection elements 7, i.e., the offset due to the dark electric charges superimposed on the actual image data D, and the offset data O read out in the offset data O readout process have the same values for the respective radiation detection elements 7.

The above-described configuration, therefore, can subtract the offset data O from the actual image data D to allow the offset due to the dark electric charges superimposed on the actual image data D and the offset data O to cancel each other out, enabling calculation of true image data D* arising only from the electric charges generated in the radiation detection elements 7 through the irradiation.

### [Processing Configuration Specific to the Present Invention etc.]

### [First Embodiment]

Next, the processing configuration specific to a first embodiment of the present invention of the radiation imaging system 100 configured as described above (see FIG. 7) is described. The behavior of the radiation imaging system 100 of the first embodiment is also described.

In the present embodiment, when the radiation imaging device 1 is taken out of the holder 56 provided at the lateral face of the radiation generating device 52 mounted on the nursing cart 51, the radiation imaging device 1 switches its power consumption mode from a sleep mode to a radiographic mode.

Alternatively, a radiation technologist E who has taken the radiation imaging device 1 out of the holder 56 may manually switch the power consumption mode of the radiation imaging device 1 to the radiographic mode by operating the switch 38, for example (see FIG. 1). Alternatively, the radiation technologist E's turning on of a power source switch 27 may change the power consumption mode of the radiation imaging device 1 to the radiographic mode.

Although the description below explains a case of using the detection method 1 as the method of irradiation-start detection, the detection method 2 may be applied with a similar configuration.

The radiation imaging device 1, whose power consumption mode has thus changed into the radiographic mode, alternately and repeatedly performs the leak data dleak readout process and the radiation detection element 7 reset process as shown in, for example, FIGS. 10 and 11. The radiation technologist E then performs positioning so that the radiation imaging device 1 is inserted between a bed B and the body of a patient B or directly placed on the body of the patient as shown in FIG. 7.

At the completion of the positioning of the radiation imaging device 1, the radiation technologist E operates the portable terminal 70 to cause a completion signal to be transmitted from the portable terminal 70 to the console 60. The radiation technologist E then moves to the radiation generating device 52 on the nursing cart 51 and operates the exposure switch 54 to irradiate the radiation imaging device 1 from the radiation source 53 through a subject.

Some kind of impact may be given to the radiation imaging device 1 when the positioning of the radiation imaging device 1 is performed, e.g., when the radiation imaging device 1 is directly placed on a patient. Such impact sometimes increases the read-out leak data dleak, as described above.

This is thought to be due to relatively large electric charges accumulating in the capacitors 18b of the readout circuits 17 in the readout IC 16 embedded in the flexible circuit board 12 (see FIGS. 6 and 8) for some reason when the impact gives instantaneous and heavy vibrations to the board 4 of the sensor panel SP and the flexible circuit board 12 (see FIG. 4). This phenomenon tends to occur, in particular, when a ceramic capacitor is used as the means to enable the thickness (reduced thickness) of the compatible-size CR.

When such impact added to the radiation imaging device 1 while the radiation imaging device 1 is being directly placed on the patient increases leak data dleak to more than threshold dleak_th, for example, the radiation imaging device 1 may falsely detect the start of irradiation although the radiation imaging device 1 is not actually irradiated.

When detecting the start of irradiation (in this case, falsely detecting the start of irradiation) in the present embodiment, the control unit 22 of the radiation imaging device 1 applies the OFF voltage to lines L1-Lx of the scan lines 5 from the gate driver 15b to shift to an electric charge accumulation state. After a lapse of a predetermined time, the control unit 22 sequentially applies the ON voltage to the scan lines 5 and performs the image data D readout process, as shown in FIG. 11.

After performing radiation imaging and reading out the image data D from the radiation detection elements 7, the control unit 22 automatically transmits the actual image data D read out from predetermined part of the radiation detection elements 7, e.g., 1/4 of all of the radiation detection elements 7, to the console 60 as the data for preview image.

The present embodiment automatically performs at least the processes so far if the start of irradiation is falsely detected.

In this case, the radiation imaging device 1 is not actually irradiated, and the actual image data D does not include any radiographed parts of the body of the patient H as a subject. The read-out actual image data D, therefore, is unnecessary data. The console 60 itself, however, cannot determine whether the transmitted data for preview image is unnecessary.

In the present embodiment, the radiation technologist E operates the portable terminal 70 at the completion the positioning of the radiation imaging device 1, inserted between a bed B and the body of a patient B or directly placed on the body of the patient B, as described above. With the completion signal transmitted from the portable terminal 70 to the console 60 as a trigger, the console 60 determines whether to allow or reject the receipt of the data for preview image received from the radiation imaging device 1.

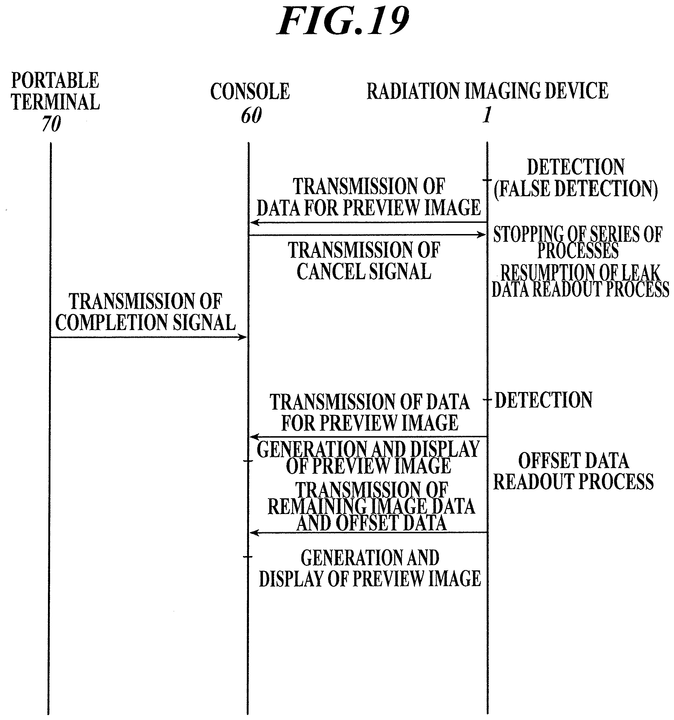

Specifically, in the present embodiment, the console 60 determines that the radiation imaging device 1 has falsely detected the start of irradiation and transmits a cancel signal to the radiation imaging device 1 when the console 60 receives the data for preview image from the radiation imaging device 1 as described above before receiving the completion signal from the portable terminal 70 (i.e., in the stage prior to receiving the completion signal), as shown in FIG. 19.

In response to receipt of the cancel signal from the console 60, the control unit 22 of the radiation imaging device 1 stops a series of processes which is being performed at that point. For example, when the offset data O readout process is being performed, the process is stopped; or when the transmission of the image data D or the offset data O has already started, the transmission process is stopped.

The leak data dleak readout process and the radiation detection element 7 reset process before the radiation imaging then resume as shown in FIGS. 10 and 11.

In this case, the control unit 22 of the radiation imaging device 1 may delete the actual image data D which has been read out and stored in the storage unit 23 (and read-out offset data O, if any) from the storage unit 23. Alternatively, the control unit 22 may set a flag to the stored actual image data D (and the offset data O), which flag indicates that the data can be overwritten, and the actual image data D etc. may be overwritten with the actual image data D etc. read out later.

At the completion of the positioning of the radiation imaging device 1, the radiation technologist E operates the portable terminal 70 and the portable terminal 70 transmits the completion signal to the console 60. When the console 60 receives the data for preview image from the radiation imaging device 1 after receiving the completion signal from the portable terminal 70, the radiation imaging device 1 is irradiated and it is determined that the radiation imaging device 1 has normally detected the start of irradiation.

In this case, the console 60 does not transmit the cancel signal to the radiation imaging device 1 but subtracts, from the data for preview image, the offset data preset for each radiation detection element 7 on the basis of the transmitted data for preview image; or subtracts, from the data for preview image, the offset data O for each radiation detection element 7 among the offset data O transmitted from the radiation imaging device 1 in the previous radiographing.

The console 60 then performs simple image processing, such as logarithmic conversion of the value obtained through the subtraction, to generate a preview image and displays the generated preview image on the display unit 61 (see FIG. 7).

As described above, the radiation imaging system and the radiation imaging device disclosed in Patent Literature 3 turn on a ready light when the device is ready to be irradiated after the TFTs 8 are turned off and the device shifts to an electric charge accumulation state. A radiation technologist E then waits for the irradiation from the radiation source 53.

Unfortunately, such a configuration causes a battery drain of the radiation imaging device and an increase in dark electric charges accumulating in the radiation detection elements 7 if a long time passes from when the radiation imaging device turns on the ready light to when the irradiation starts. This causes problems such as a poor S/N ratio of the read-out image data D.

In contrast, in the radiation imaging system 100 of the present embodiment, the radiation imaging device 1 itself detects and determines the start of irradiation. The radiation imaging device 1 starts the actual image data D readout process a predetermined time after the detection of the start of irradiation, for which predetermined time the radiation imaging device 1 is in the electric charge accumulation state, as shown in, for example, FIG. 11.

This surely prevents the electric charge accumulation state from becoming abnormally long and surely prevents the battery 24 from overconsuming power (see FIG. 5, for example). Further, this prevents the effective accumulation time Tac from becoming longer than is necessary, preventing problems such as a poor S/N ratio of the read-out image data D due to the increase in dark electric charges accumulating in the radiation detection elements 7.

The radiation imaging device 1 detects the start of irradiation on the basis of the leak data dleak (or the image data d for irradiation-start detection) read out before the radiation imaging. An impact on the radiation imaging device 1 at the time of the positioning of the radiation imaging device 1, for example, may increase the read-out leak data dleak, as described above.

This may cause the radiation imaging device 1 to falsely detect the start of irradiation. The console 60 itself, however, cannot determine whether the data for preview image received from the radiation imaging device 1 is based on a false detection or not.

In view of the above, the console 60 of the present embodiment determines whether the data for preview image received from the radiation imaging device 1 is based on a false detection before or after the completion of the positioning of the radiation imaging device 1, as described above. The positioning, which is performed by a radiation technologist E is, for example, insertion of the radiation imaging device 1 between a bed B and the body of a patient B or direct placement of the radiation imaging device 1 on the body of the patient B.

Specifically, the data for preview image transmitted before the completion of the positioning of the radiation imaging device 1 by the radiation technologist E can be considered to be based on a false detection because the radiation imaging device 1 cannot be irradiated before the completion of the positioning of the radiation imaging device 1.

The radiation imaging device 1 is less likely to be newly subject to a heavy impact after the completion of the positioning of the radiation imaging device 1. The data for preview image transmitted after the completion of the positioning of the radiation imaging device 1, therefore, can be considered to be based on a normal detection, namely based on the actual image data D obtained through a proper radiographing of a subject.

When a heavy impact is given to the radiation imaging device 1 after the positioning, the radiation technologist E can recognize the impact and simply needs to wait for a several seconds without performing irradiation and check whether a preview image is displayed on the display unit 61 of the console 60. When no preview image is displayed, the radiation technologist E can determine that no false detection has occurred and can operate the exposure switch 54 (see FIG. 7) for radiographing.

When a preview image is displayed, the radiation technologist E can determine that a false detection has occurred, and make an input for cancelling directly to the console 60 or through the portable terminal 70 so that the cancel signal is transmitted from the console 60 to the radiation imaging device 1. In this case, the radiation imaging device 1 stops the process which is being performed at that point as described above. For example, when the offset data O readout process is being performed, the process is stopped; or when the transmission of the actual image data D or the offset data O has already started, the transmission process is stopped. The radiation imaging device 1 then resumes the leak data dleak readout process and the radiation detection element 7 reset process before the radiation imaging. The radiation technologist E can then operate the exposure switch 54 (see FIG. 7) for radiographing.

As described above, the radiation imaging device 1 of the radiation imaging system 100 in accordance with the present embodiment performs the leak data dleak readout process and the readout process to read out the image data d for irradiation-start detection before the radiation imaging, and detects the start of irradiation on the basis of the read-out leak data dleak etc.

This prevents a time for which the TFTs 8 are in the OFF state after the detection of the start of irradiation from becoming too long, surely preventing overconsumption of the power of the battery 24 (see FIG. 5, for example). Further, the radiation imaging device 1 can surely prevent problems such as a poor S/N ratio of the read-out image data D due to increase in dark electric charges accumulating in the radiation detection elements 7 while the TFTs 8 are in the OFF state (namely, during the effective accumulation time Tac).

Further, the console 60 of the radiation imaging system 100 in accordance with the present embodiment determines that the radiation imaging device 1 has falsely detected the start of irradiation and transmits the cancel signal to the radiation imaging device 1 when the console 60 receives the data for preview image from the radiation imaging device 1 before receiving the completion signal from the portable terminal 70 (i.e., before the radiation technologist E completes the positioning of the radiation imaging device 1). The radiation imaging device 1 then stops the process which is being performed at that point and resumes the leak data dleak readout process or the readout process to read out the image data d for irradiation-start detection before the radiation imaging.

When the console 60 receives the data for preview image from the radiation imaging device 1 after receiving the completion signal from the portable terminal 70 upon completion of the positioning of the radiation imaging device 1 by the radiation technologist E, the console 60 determines that the radiation imaging device 1 has normally detected the start of irradiation, and generates a preview image on the basis of the data for preview image to display it on the display unit 61.

Hence, the console 60 can make a determination of a false detection properly on the basis of whether the completion signal is transmitted from the portable terminal 70, i.e., whether the radiation technologist E has completed the positioning of the radiation imaging device 1. Specifically, the console 60 can accurately determine that data for preview image is based on a false detection and can make the radiation imaging device 1 stop a series of processes when the console 60 receives the data for preview image before receiving the completion signal from the portable terminal 70 (i.e., before the radiation technologist E completes the positioning of the radiation imaging device 1). The console 60 can then bring the radiation imaging device 1 to the state to perform the leak data dleak readout process and the readout process to read out the image data d for irradiation-start detection before the radiation imaging.

This eliminates the need for the radiation technologist E to wait for the completions of the readout processes of the actual image data D and the offset data O based on a false detection and enables the radiation technologist E to operate the exposure switch 54 immediately for a proper radiographing. The entire radiation imaging system 100 including the radiation imaging device 1 is thus convenient for the radiation technologist E.

The console 60 determines that the radiation imaging device 1 has normally detected the start of irradiation and generates a preview image on the basis of the data for preview image to display it on the display unit 61. In response to receipt of the rest of the actual image data D and the offset data O from the radiation imaging device 1 as shown in FIG. 19, the console 60 performs predetermined image processing, such as offset correction, gain correction, defective pixel correction, and gradation processing, on the actual image data D as described above, to generate a radiation image (medical image) and display it on the display unit 61.

When the radiation technologist E who has checked a preview image judges that a subject is not at a normal position in the captured radiation image and inputs, to the console 60, the information of not approving the preview image (i.e., the information of disapproval), the console 60 makes the radiation imaging device 1 discard the actual image data D, stop a series of processes which is being performed at that point, and resume the leak data dleak readout process and the radiation detection element 7 reset process before the radiation imaging for re-radiographing.

These processes are performed as an ordinary re-radiographing process originally provided for the console 60, unlike the cancel process to be performed by the console 60 specific to the present invention.

### [Modifications]

Modifications of the configuration of the radiation imaging system 100 of the first embodiment are described below. The modifications are applied to a second embodiment, described later, as appropriate if possible.

### [Modification 1]

In the case where the portable terminal 70 includes a display screen 71 as shown in FIG. 7, the portable terminal 70 which has received a generated preview image from the console 60 can display the preview image on the display screen 71.

In this case, when the console 60 receives the data for preview image based on a false detection from the radiation imaging device 1 before receiving the completion signal from the portable terminal 70, the console 60 transmits the cancel signal to the radiation imaging device 1 as described above, and at the same time, performs a decimation process on the data for preview image, for example, to generate a preview image for the portable terminal 70. The console 60 can transmit the generated preview image for the portable terminal 70 to the portable terminal 70.

With such a configuration, the data for preview image based on the false detection does not include a radiographed subject, and the preview image displayed on the display screen 71 of the portable terminal 70 is an abnormal image, as it were. The radiation technologist E, who checks such a preview image, can surely recognize that the radiation imaging device 1 has falsely detected the start of irradiation for some reason such as an impact.

When the radiation imaging device 1 normally detects the start of irradiation and transmits normal actual image data D, i.e., the data for preview image based on the actual image data D including a radiographed subject, to the console 60 as described above, the radiation technologist E checks the preview image displayed on the display unit 61 of the console 60 to approve or disapprove the preview image. The radiation technologist E can input approval or disapproval of the preview image through the portable terminal 70.

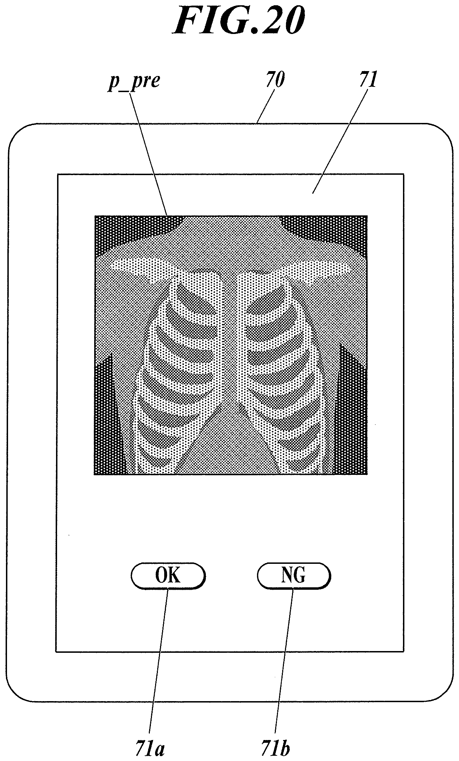

Specifically, in response to receipt of the generated preview image from the console 60 as described above, the portable terminal 70 displays a preview image p_pre, an "OK" button 71a, and an "NG" button 71b on the display screen 71 as shown in FIG. 20 on the basis of the received preview image.

The radiation technologist E then confirms the displayed preview image p_pre. When approving the preview image p_pre, the radiation technologist E touches the "OK" button 71a to input approval, and otherwise the radiation technologist E touches the "NG" button 71b to input disapproval. The portable terminal 70 then transmits the signal corresponding to the input approval or disapproval to the console 60. In response to the signal corresponding to the approval or disapproval received from the portable terminal 70, the console 60 accordingly performs the same processes as in the case where approval or disapproval is directly input to the console 60 itself.

Such a configuration eliminates the need for the radiation technologist E to stay around the nursing cart 51 (see FIG. 7), on which the console 60 is mounted, after the radiographing to confirm the preview image p_pre displayed on the display unit 61 of the console 60. The radiation technologist E can confirm the preview image p_pre displayed on the display screen 71 of the portable terminal 70 while performing another process, such as changing the position of the radiation imaging device 1 directly placed on the patient H, for a next radiographing.

This enables a smooth radiographing when multiple radiographs are continuously taken for the patient H. The radiation imaging system 100 including the radiation imaging device 1 is thus more convenient for the radiation technologist E.

### [Modification 2]

It is preferable that the radiation imaging device 1 falsely detects the start of irradiation as little as possible from the time when the radiation technologist E completes the positioning of the radiation imaging device 1 to when the completion signal is transmitted from the portable terminal 70.

In view of this, the threshold dleak (see FIG. 12) and the threshold dth to be used to detect the start of irradiation for the radiation imaging device 1 can be kept to values higher than normal thresholds dleak_th and dth set through the methods shown in FIGS. 13 and 14 before the completion signal is transmitted from the portable terminal 70, for example. This prevents the read-out leak data dleak etc., which exhibits a high value due to, for example, an impact, from exceeding the threshold dleak_th as much as possible.

Specifically, in response to receipt of the completion signal from the portable terminal 70 as described above, the console 60 transmits a signal representing receipt of the completion signal to the radiation imaging device 1. The completion signal itself may be transferred to the radiation imaging device 1.

The control unit 22 of the radiation imaging device 1 sets the threshold dleak_th etc. to a value obtained by adding a value twenty times the standard deviation σ shown in FIG. 13, for example, to the average da of the leak data dleak to detect the start of irradiation on the basis of the set threshold dleak before receiving the signal representing receipt of the completion signal from the console 60.

After receiving the signal representing receipt of the completion signal from the console 60, the control unit 22 sets the threshold dleak_th etc. to a value obtained by adding a value eight times the standard deviation σ shown in FIG. 13, for example, to the average da of the leak data dleak to detect the start of irradiation on the basis of the set threshold dleak.

This configuration allows the control unit 22 of the radiation imaging device 1 to accurately detect the start of irradiation on the basis of the normal threshold dleak_th etc., as described above, after receiving the signal representing receipt of the completion signal from the console 60.

Setting the threshold dleak_th etc. to a higher value before the receipt of the signal representing receipt of the completion signal from the console 60 can surely reduce the possibility that the read-out leak data dleak etc. exceeds the threshold dleak_th etc. if the leak data dleak etc. of a high value is read out due to an impact, for example.

The radiation imaging device 1 can thus surely reduce the frequency of a false detection of the start of irradiation before the radiation technologist E completes the positioning of the radiation imaging device 1.

### [Modification 3]

The above-described embodiments explain the case where the power consumption mode automatically changes from the sleep mode to the radiographic mode when the radiation imaging device 1 is taken out of the holder 56 on the nursing cart 51 (see FIG. 7) so that the leak data dleak readout process (or the readout process to read out the image data d for irradiation-start detection) before the radiation imaging starts.

The configuration is not necessarily limited thereto. For example, the radiation imaging device 1 may be in a power-off state when held in the holder 56 on the nursing cart 51. The radiation imaging device 1 may be powered on in response to the radiation technologist E's operation of the power source switch 37 (see FIG. 1) after he/she takes the radiation imaging device 1 out of the holder 56.

In such a case, the radiation technologist E may forget to turn on the radiation imaging device 1 after taking it out of the holder 56. That is, the radiation technologist E may, for example, directly place the radiation imaging device 1 on the body of a patient H (see FIG. 7) and irradiate the OFF-state radiation imaging device 1. This causes a wrong exposure, leading to an irradiation for nothing.

Such a wrong exposure wastefully exhausts the radiation source 53 and requires another radiographing. As a result, the patient H is to be irradiated again, which may lead to an increase in an exposure dose.

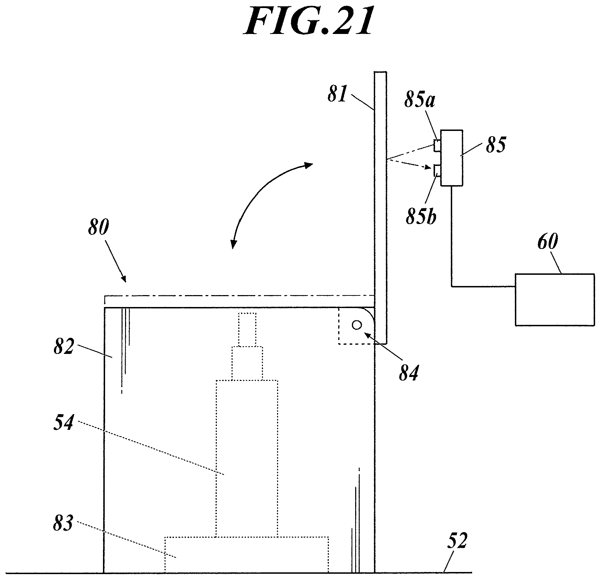

In order to prevent the radiation technologist E from operating the exposure switch 54 without turning on the radiation imaging device 1, the nursing cart 51 may include a wrong exposure prevention unit 80 as shown in FIG. 21, for example.

A cover in the shape of a casing surrounding the exposure switch 54 as shown in FIG. 21 may be used as the wrong exposure prevention unit 80. The wrong exposure prevention unit 80 includes a cover 81 and a support plate 82, for example. The cover 81 may be opened and closed relative to the support plate 82.

The support plate 82 may consist of two plate members arranged so as to stand in parallel with each other at the both sides of the exposure switch 54 held on the holder 83. Alternatively, the support plate 82 may be arranged so as to surround the exposure switch 54 held on the holder 83 on three or four sides. The cover 81 is attached to the support plate 82 so as to open and close relative to the support plate 82 with a hinge structure 84 provided at one end of the upper part of the support plate 82 in such a way that the cover 81 can open and close.

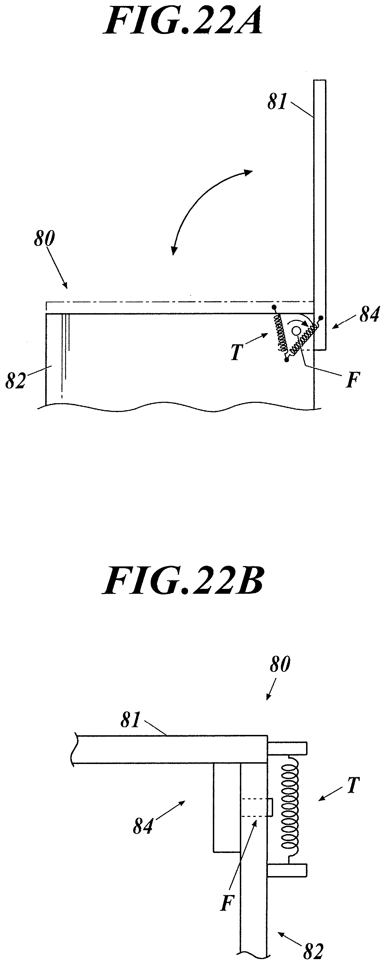

The hinge structure 84 of the wrong exposure prevention unit 80 may be provided with a tumbler spring T as shown in FIGS. 22A and 22B, for example. The tumbler spring T forces the cover 81 to be closed, but forces the cover 81 to be opened when the radiation technologist E performs an opening operation of the cover 81 around the rotation axis F to a predetermined position or wider.

Providing the wrong exposure prevention unit 80 around the exposure switch 54 as described above requires the radiation technologist E to perform the opening operation for the wrong exposure prevention unit 80 to operate the exposure switch 54 for irradiation. The radiation technologist E can be reminded of powering on the radiation imaging device 1 when performing the opening operation for the wrong exposure prevention unit 80. This can prevent a wrong exposure of the radiation imaging device 1 which is in the power-off state.

In the case where the wrong exposure prevention unit 80 is provided around the exposure switch 54, the radiation technologist E's opening operation of the cover 81 of the wrong exposure prevention unit 80 can be regarded as a kind of cue indicating that the radiation imaging device 1 is to be irradiated. The radiation imaging device 1 is not irradiated before the cue.

The power consumption mode of the radiation imaging device 1 is the sleep mode before the radiation technologist E performs the opening operation for the wrong exposure prevention unit 80, for example. When the radiation technologist E performs the opening operation for the wrong exposure prevention unit 80, the radiation imaging device 1 changes its power consumption mode to the radiographic mode to start a process, such as the leak data dleak readout process before the radiation imaging.

Such a configuration can prevent the control unit 22 of the radiation imaging device 1 from falsely detecting the start of irradiation even when the radiation imaging device 1 is subject to an impact at least while the power consumption mode of the radiation imaging device 1 is the sleep mode. In order to achieve this, the following configuration may be applied.

Specifically, an optical detector 85 including a light-emitting element 85a and a light-receiving element 85b is disposed so that the light emitted from the light-emitting element 85a is reflected by the open-state cover 81 to enter the light-receiving element 85b, as shown in FIG. 21. The optical detector 85 is electrically connected to the console 60. When detecting the opening of the cover 81, the optical detector 85 transmits an opening signal to the console 60.

The support plate 82 of the wrong exposure prevention unit 80 may be provided with a detector (not shown) at, for example, the upper end part of the support plate 82. The detector detects the contact with the cover 81. When the cover 81 and the support plate 82 are out of contact from each other, it is determined that the cover 81 is opened and the detector transmits the opening signal to the console 60.

upon determining that the radiation technologist E has performed the opening operation for the wrong exposure prevention unit 80 and the exposure switch 54 has become operable on the basis of the opening signal from the optical detector 85, the console 60 transmits a wake-up signal to the radiation imaging device 1.

In response to receipt of the wake-up signal from the console 60, the radiation imaging device 1 changes its power consumption mode from the sleep mode to the radiographic mode to start the leak data dleak readout process and the readout process to read out the image data d for irradiation-start detection before the radiation imaging.

Such a configuration can surely prevent the control unit 22 of the radiation imaging device 1 from falsely detecting the start of irradiation even when the radiation imaging device 1 is subject to an impact at least before the radiation technologist E performs the opening operation for the wrong exposure prevention unit 80 to operate the exposure switch 54, i.e., while the power consumption mode of the radiation imaging device 1 is the sleep mode.

After that, as already described in the above embodiment, the console 60 transmits the cancel signal to the radiation imaging device 1 when the console 60 receives the data for preview image from the radiation imaging device 1 based on a false detection before receiving the completion signal from the portable terminal 70; and the console 60 accepts the data for preview image transmitted from the radiation imaging device 1 after receiving the completion signal from the portable terminal 70.

As already described in Modification 2, the threshold dleak_th and the threshold dth may be switched from higher values to normal values upon receipt of the completion signal from portable terminal 70.

In Modification 3, the radiation technologist E may forget to turn on the radiation imaging device 1 when performing the opening operation for the wrong exposure prevention unit 80, and may turn on the radiation imaging device 1 after performing the opening operation for the wrong exposure prevention unit 80. In this case, the power consumption mode of the radiation imaging device 1 is the sleep mode immediately after the device 1 is turned on, but quickly changes to the radiographic mode because the wake-up signal has already been transmitted from the console 60 at this point.

### [Second Embodiment]

In the first embodiment, the detection of the start of irradiation with the radiation imaging device 1 before the timing when the console 60 receives the completion signal from the portable terminal 70 in response to the radiation technologist E's operation of the portable terminal 70 at the completion of the positioning of the radiation imaging device 1 is determined as a false detection; while the detection of the start of irradiation with the radiation imaging device 1 after the timing is determined as a normal detection.

Further considering the configuration to minimize the occurrence of the false detection before the console 60 receives the completion signal from the portable terminal 70 as in Modifications 2 and 3, the power consumption mode of the radiation imaging device 1 can be the sleep mode so as not to allow the detection of the start of irradiation before the console 60 receives the completion signal from the portable terminal 70.

In this case, the power consumption mode of the radiation imaging device 1 may be changed to the radiographic mode at the timing when the console 60 receives the completion signal from the portable terminal 70 at the completion of the positioning of the radiation imaging device 1.

Such a configuration prevents a false detection of the start of irradiation, even when the radiation imaging device 1 is subject to an impact, before the console 60 receives the completion signal from the portable terminal 70 at the completion of the positioning of the radiation imaging device 1 because the leak data dleak readout process etc. has not been performed.

This can surely prevent the radiation imaging device 1 from falsely detecting the start of irradiation before the console 60 receives the completion signal from the portable terminal 70, even if the radiation imaging device 1 is subject to an impact.

More specifically, the power consumption mode of the radiation imaging device 1 is switchable between the radiographic mode and the sleep mode as described above. When the power source switch 37 of the radiation imaging device 1 (see FIG. 1) is turned on, the radiation imaging device 1 shifts to the sleep mode, for example.

In this case, unlike the first embodiment, the radiation imaging device 1, whose power consumption mode is the sleep mode, does not transmit the data for preview image based on a false detection before the console 60 receives the completion signal from the portable terminal 70. The console 60, therefore, does not transmit the cancel signal to the radiation imaging device 1.

The radiation technologist E performs positioning of the radiation imaging device 1 by, for example, directly placing the radiation imaging device 1 in the sleep mode on the body of the patient H (see FIG. 7).

At the completion of the positioning of the radiation imaging device 1, the radiation technologist E operates the portable terminal 70 so that the completion signal is transmitted from the portable terminal 70 to the console 60. In response to receipt of the completion signal, the console 60 transmits the wake-up signal to the radiation imaging device 1. In response to receipt of the wake-up signal from the console 60, the radiation imaging device 1 changes its power consumption mode from the sleep mode to the radiographic mode to start the leak data dleak readout process, for example.

After that, as described in the first embodiment, the control unit 22 of the radiation imaging device 1 detects the start of irradiation on the basis of the read-out leak data dleak etc. (see FIGS. 11 and 17), shifts to the electric charge accumulation state, and then reads out the actual image data D from the radiation detection elements 7.

Part of the read-out actual image data D is then transmitted to the console 60 as the data for preview image. The offset data O readout process is performed as shown in FIG. 18, for example, and the rest of the actual image data D and the offset data O are transmitted to the console 60.

In response to receipt of the data for preview image from the radiation imaging device 1, the console 60 generates a preview image p_pre on the basis of the data for preview image to display it on the display unit 61. When the radiation technologist E disapproves the preview image p_pre, the console 60 makes the radiation imaging device 1 discard the actual image data D, stop a series of processes which is being performed at that point, and resume the leak data dleak readout process etc. before the radiation imaging for re-radiographing.

When the preview image p_pre is approved, the console 60 generates a radiation image on the basis of the received actual image data D and the offset data O, and displays the generated radiation image on the display unit 61.

As described above, the radiation imaging system of the present embodiment changes the power consumption mode of the radiation imaging device 1 from the sleep mode to the radiographic mode when the console 60 receives the completion signal from the portable terminal 70 at the completion of the positioning of the radiation imaging device 1 by the radiation technologist E.

This can surely prevent the radiation imaging device 1, whose power consumption mode is the sleep mode, from falsely detecting the start of irradiation even if the radiation imaging device 1 is subject to an impact while the radiation technologist E is performing positioning of the radiation imaging device 1 by, for example, directly placing the radiation imaging device 1 on the body of the patient H.

After the console 60 receives the completion signal from the portable terminal 70 in response to the completion of the positioning of the radiation imaging device 1, the power consumption mode of the radiation imaging device 1 changes to the radiographic mode. Thus, the radiation imaging device 1 can surely detect the start of irradiation on the basis of the read-out the leak data dleak and the image data d for irradiation-start detection when irradiated.

Further, after the completion of the positioning of the radiation imaging device 1 and before the console 60 receives the completion signal from the portable terminal 70, the power consumption mode of the radiation imaging device 1 is in the sleep mode, namely a power-saving mode. This can prevent the battery 24 of the radiation imaging device 1 (see FIG. 5, for example) from wasting electricity during the time.

In the case of sequential radiographing, e.g., taking multiple radiation images of one patient in various postures, the power consumption mode of the radiation imaging device 1 is brought into the sleep mode after the completion of the image data D readout process etc. by the radiation imaging device 1 for one radiographing. The power consumption mode is then shifted to the radiographic mode again for the next radiographing in response to the completion signal from the portable terminal 70 after the completion of the positioning of the radiation imaging device 1 as a trigger.

The switching of the imaging mode described above may also be applied to an additional radiographing. In this case, the power consumption mode of the radiation imaging device 1 may be changed from the sleep mode to the radiographic mode when radiographing order information to instruct an additional radiation imaging is newly input, or the power consumption mode may be changed to the radiographic mode in response to the completion signal transmitted from the portable terminal 70 to the console 60 as a trigger.

In the case where the radiation generating device 52 includes an exposure switch 54 to be operated in two steps (see FIG. 7) and an operation of the exposure switch can be input to the console 60 as a trigger signal, the following configuration may be employed.

A first-step pressing operation of the exposure switch 54 for two-step operation starts the radiation source 53, and a second-step pressing operation makes an irradiation. Even if the first- and second-step operations are performed in succession, a mechanical resistance provided against the change between the first and second pressing operations allows a predetermined time difference (about one second, in general) between the first and second pressing operations. The mechanical resistance allows a stable radiation source within the time difference.

The detection of the first-step operation of the exposure switch 54 may be a trigger of the change of the power consumption mode of the radiation imaging device 1 from the sleep mode to the radiographic mode. In this case, the time difference is sufficient for the completion of preliminary preparations for a series of reset processes etc. required for the mode change. The detection of the timing of the actual radiation start, therefore, is not influenced at all.

Preparations for radiographing such as positioning of the radiation imaging device 1 on the body of a patient have already been completed by the time when a radiographer, such as a radiation technologist, operates the exposure switch 54. The radiation imaging device 1, therefore, is not subject to an impact and the like.

This eliminates the need to set the threshold dleak (see FIG. 12) and the threshold dth, which are used to detect the start of irradiation by the radiation imaging device 1 as described above, to be higher than normal thresholds dleak_th and dth, which are set through the methods shown in FIGS. 13 and 14. This also eliminates the need for the transmission operation itself of the completion signal from the portable terminal 70 to the console 60 to prevent a false detection.

The explanations for the first and second embodiments are made based on the assumption that the radiation imaging device 1 and the portable terminal 70 correspond one-to-one with the access point 55 provided on the nursing cart 51 as shown in FIG. 7. In other words, it is assumed that the radiation imaging device 1 and the portable terminal 70 are used exclusively for the radiation imaging system 100 constructed on the nursing cart 51.

In this case, the radiation imaging device 1 and the portable terminal 70 on which the SSID of the access point 55 is registered in advance can communicate with the console 60 and transmit data such as the actual image data D to the console 60 through the access point 55 without requiring the SSID to be registered again.

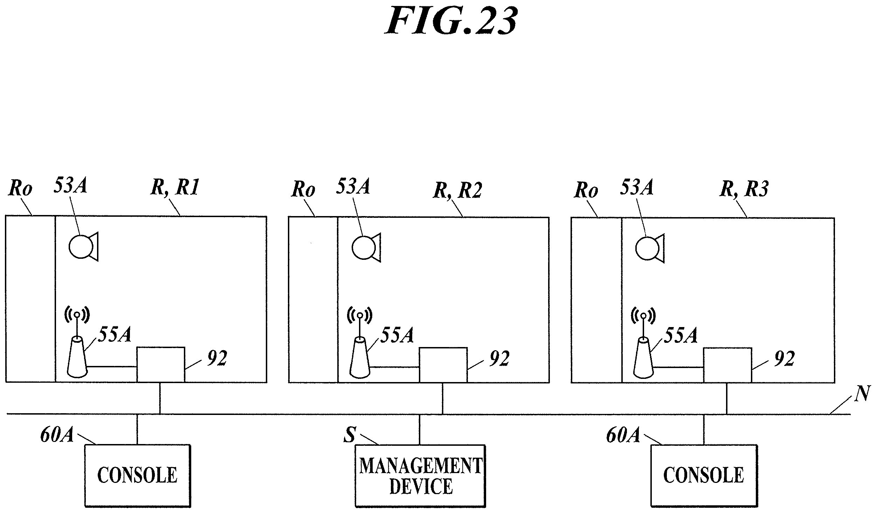

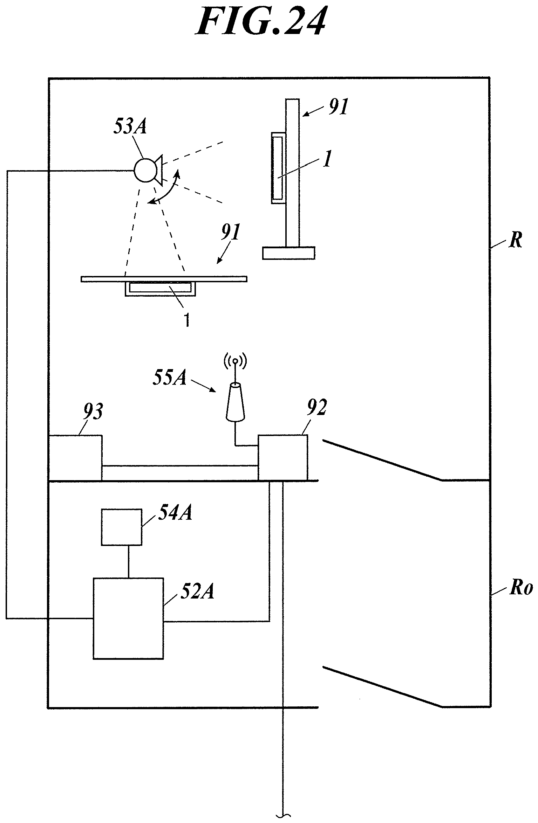

Some facilities such as hospitals using the radiation imaging device 1 and the like have radiographic rooms R for radiation imaging as shown in FIGS. 23 and 24. The radiation imaging device 1 and the portable terminal 70 may be used both for the radiation imaging system 100 constructed on the nursing cart 51 as described above and in the radiographic rooms R.

First, such radiographic rooms R are briefly described below. The devices having the same functions as the devices in the radiation imaging system 100 shown in FIG. 7 are indicated by the reference numerals for the radiation imaging system 100 with "A" added.

FIG. 23 shows a plurality of radiographic rooms R (R1-R3) connected with a plurality of consoles 60A and a management device S via a network N. The management device S constituted of a server computer, for example, manages which radiation imaging device 1 exists in each radiographic room R, for example. Specifying any one radiographic room R on a console 60A associates the console 60A one-to-one with the radiographic room R.

As shown in FIG. 24, a radiographic room R includes a radiation source 53A, Bucky devices 91 in which the radiation imaging devices 1 are respectively loaded, and a relay 92 to which an access point 55A is connected, for example. A front room Ro includes a radiation generating device 52A having an exposure switch 54A. The relay 92 relays communication between the devices in the radiographic room R or the front room Ro and a console 60A.

Although FIG. 24 shows both a standing radiograph Bucky device and a supine radiograph Bucky device as the Bucky devices 91, there are many cases where only one of the Bucky devices is provided.

The relay 92 is connected with a cradle 93. The cradle 93 typically stores the inserted radiation imaging device 1 and charges the battery 24 of the radiation imaging device 1 (see FIG. 5, for example). In this embodiment, the cradle 93 further functions as a registration means for the radiation imaging device 1.

Specifically, the radiation imaging device 1 brought in the radiographic room R and inserted in the cradle 93 by a radiation technologist E transmits a cassette ID, which is the identification information of the radiation imaging device 1, to the management device S through the cradle 93 and the relay 92.

The SSID of the access point 55 provided in the radiographic room R is registered for the radiation imaging device 1, which is inserted in the cradle 93, from the management device S and the cradle 93 in order to prevent interference with another radiographic room R.

In performing radiation imaging with the radiation imaging device 1 for which the SSID has been thus registered and which is loaded in the Bucky device 91, for example, the radiation source 53A may emit radial rays while the radiation imaging device 1 and the radiation generating device 52A are synchronized with each other through a console 60A for radiographing, rather than the radiation imaging device 1 itself detects the start of irradiation as in the above-described embodiments.

This eliminates the need for the radiation technologist E to operate the portable terminal 70 to notify the console 60A of the completion of the positioning of the radiation imaging device 1 as in the above-described embodiments.

In the case where a patient H (see FIG. 7) cannot get up from the bed B, the patient H may be carried along with the bed B to a radiographic room R, where a portable radiation source, for example, is brought for radiographing.

In such a case, the radiographing method in accordance with the present disclosure, which has been described in the embodiments above, is performed with the use of the console 60A provided in the facility and the access point 55A (see FIG. 23) in the radiographic room R. In this case, not registering the SSID of the access point 55A of the radiographic room R on the portable terminal 70 fails to allow the communications between the portable terminal 70 and the console 60A through the access point 55A.

In view of this, a radiographic room R and the SSID of the access point 55A provided in the radiographic room R are stored in the portable terminal 70 in advance, for example, with the SSID and the radiographic room R being associated with each other for each radiographic room R. A radiation technologist E is allowed to operate the portable terminal 70 when he/she brings the portable terminal 70 into a radiographic room R. At this time, the display screen 71 of the portable terminal 70 displays a message such as "In which radiographic room do you use this terminal?" to allow the radiation technologist E to select a radiographic room R where the portable terminal 70 is to be used.

The portable terminal 70 may be configured to read out the SSID of the access point 55A associated with the radiographic room R selected by the radiation technologist E and to register the SSID. Such a configuration enables, afterwards, the portable terminal 70 to communicate with the console 60A through the access point 55A provided in the radiographic room R and surely enables radiation imaging in the method according to the present disclosure described in the embodiments when the radiation imaging is performed in the radiographic room R.

In this case, a preview image for the portable terminal 70 may be generated through a decimation process based on the generated preview image p_pre and may be transmitted from the console 60A to the portable terminal 70 carried by the radiation technologist E in the radiographic room R so that the portable terminal 70 displays the preview image p_pre on its display screen 71, as described in the Modification 1. This allows the radiation technologist E to view the preview image p_pre displayed on the display screen 71 of the portable terminal 70 in the radiographic room R without getting out of the radiographic room R to go to the console 60A.

This enables very easy checking of the preview image p_pre i.e., the determination of approval or disapproval described above, allowing the radiation imaging system to be very convenient for the radiation technologist E.

In the case where the radiation imaging device 1 is used while loaded in the Bucky device 91 so that the radiation imaging device 1 itself detects the start of irradiation, the radiation imaging device 1 is easily subject to a heavy impact at the time when a radiation technologist E loads the radiation imaging device 1 in the Bucky device 91. There is a possibility that the radiation imaging device 1 falsely determines the start of irradiation due to the impact. It should naturally be understood that the above-described process (i.e., cancelling operation) may be performed in the radiographic room through the portable terminal 70.

It should naturally be understood that the present invention is not limited to the above-described embodiments.

### INDUSTRIAL APPLICABILITY

The present invention is applicable in the field of performing radiation imaging and particularly in the field of medicine.

### REFERENCE NUMERALS

- 1: radiation imaging device
- 5: scan line
- 6: signal line
- 7: radiation detection element
- 8: TFT (switch section)
- 15: scan driving unit
- 17: readout circuit
- 22: control unit
- 24: battery
- 41: antenna device (communication unit)
- 52: radiation generating device
- 53: radiation source
- 54: exposure switch
- 60: console
- 70: portable terminal
- 71: display screen
- 80: wrong exposure prevention unit
- 100: radiation imaging system
- D: image data
- d: image data for irradiation-start detection
- dleak: leak data
- dleak_th: threshold
- dth: threshold
- O: offset data
- p_pre: preview image
- q: electric charge
- r: small region

## Claims

1. A radiation imaging system (100) comprising:
a portable radiation imaging device (1) including:
a plurality of scan lines (5);
a plurality of signal lines (6) disposed to intersect with the scan lines (5);
a plurality of radiation detection elements (7) two-dimensionally disposed in small regions (r) formed by the scan lines (5) and the signal lines (6);
a scan driving unit (15) which applies ON voltage or OFF voltage to the scan lines (5);
a plurality of switch sections (8) which are connected to the scan lines (5) and release electric charges accumulated in the radiation detection elements (7) to the signal lines when the ON voltage is applied;
a readout circuit (17) which convert the electric charges released from the radiation detection elements (7) to actual image data (D) and reads out the actual image data (D);
a control unit (22) which performs a leak data readout process before radiation imaging, the leak data readout process being a process to convert the electric charges leaking from the radiation detection elements via the switch sections to leak data with the scan driving unit applying the OFF voltage to the scan lines so that the switch sections are in an OFF state, the control unit detecting a start of irradiation when the read-out leak data exceeds a threshold; and
a communication unit (41) with which the image data (D) is transmitted to an external device (60);
a radiation generating device (52) which control a radiation source (53) to irradiate the radiation imaging device (1);
a console (60) which generate a preview image (p pre) and a radiation image on the basis of the actual image data (D) received from the radiation imaging device (1); and
a portable terminal (70) which transmit a completion signal to the console (60) when the portable terminal (70) receives an input of information indicating that positioning of the radiation imaging device (1) has been completed,
wherein, when the control unit (22) of the radiation imaging device (1) detects the start of irradiation, the control unit (22) change to an electric charge accumulation state and then allows the actual image data (D) to be read out from the radiation detection elements (7) to transmit part of the read-out actual image data (D) to the console (60) as data for preview image, the electric charge accumulation state being a state in which the scan driving unit (15) applies the OFF voltage to the scan lines (5) to accumulate electric charges generated due to the irradiation in the radiation detection elements (7);
wherein, when the console (60) receive the data for preview image from the radiation imaging device (1) before receiving the completion signal from the portable terminal (70), the console (60) transmits a cancel signal to the radiation imaging device (1), stops a process which is being performed by the radiation imaging device (1), and makes the leak data (d) readout for irradiation start detection process before the radiation imaging resumes; and
wherein, when the console (60) receive the data for preview image from the radiation imaging device (1) after receiving the completion signal from the portable terminal (70), the console (60) generates the preview image (p pre) on the basis of the data for preview image.

2. The radiation imaging system (100) according to claim 1,
wherein the radiation imaging device (1) perform an offset data (O) readout process to read out offset data (O) equivalent to an offset resulting from dark electric charges superimposed on the actual image data (D) and transmits the offset data (O) along with rest of the image data (D) to the console (60) after the radiation imaging device (1) transmits the data for preview image; and
wherein the radiation imaging device (1) stops reading out and transmitting the offset data (O) in response to receipt of the cancel signal from the console (60) and resumes the leak data readout process before the radiation imaging or the image data readout process to read out the image data for irradiation-start detection before the radiation imaging.

3. The radiation imaging system according to any one of claims 1 or 2,
wherein the console transmits a signal representing receipt of the completion signal to the radiation imaging device in response to receipt of the completion signal from the portable terminal; and
wherein, until receiving the signal representing receipt of the completion signal, the control unit of the radiation imaging device keeps the threshold to a value higher than a normal threshold after the control unit receives the signal representing receipt of the completion signal.

4. The radiation imaging system (100) according to any one of claims 1 to 3,
wherein the console (60) transmit the generated preview image (p pre) to the portable terminal (70); and
wherein the portable terminal (70) includes a display screen (71) on which the preview image (p pre) transmitted from the console (60) is displayed.

5. The radiation imaging system according to claim 4,
wherein, upon transmission of the cancel signal to the radiation imaging device, the console generates the preview image on the basis of the data for preview image and transmits the generated preview image to the portable terminal.

6. The radiation imaging system according to claim 4 or claim 5,
wherein approval or disapproval of the preview image (p pre) can be input through the portable terminal (70) to be transmitted to the console (60).

7. The radiation imaging system (100) according to any one of claims 1 to 6,
wherein the radiation generating device (52) includes:
an exposure switch (54) with which the start of irradiation is instructed to the radiation source (53), and
a wrong exposure prevention unit (80) to prevent the exposure switch (54) from being operated, the wrong exposure prevention unit (80) capable of opening and closing;
wherein the console (60) is configured to change a power consumption mode of the radiation imaging device (1) from a sleep mode to a radiographic mode when the console (60) detects that an opening operation of the wrong exposure prevention unit (80) has been performed and that the exposure switch (54) has been put into an operable state, the sleep mode being a mode in which radiation imaging is not performed with electric power supplied from a battery (24) only to a necessary functional part including at least the communication unit (41), and the radiographic mode being a mode in which the electric power is supplied to functional parts including at least the scan driving unit(15), the readout circuit (17), and the control unit (22) so that the radiation imaging can be performed; and
wherein, when the power consumption mode is changed to the radiographic mode, the control unit (22) of the radiation imaging device (1) starts the leak data readout process before the radiation imaging or the image data readout process to read out the image data for irradiation-start detection before the radiation imaging.

8. A radiation imaging system (100) comprising:
a portable radiation imaging device (1) including:
a plurality of scan lines (5);
a plurality of signal lines (6) disposed to intersect with the scan lines;
a plurality of radiation detection elements two-dimensionally disposed in small regions formed by the scan lines (5) and the signal lines;
a scan driving unit (15) which applies ON voltage or OFF voltage to the scan lines (5);
a plurality of switch sections (8) which are connected to the scan lines (5) and release electric charges accumulated in the radiation detection elements (7) to the signal lines (6) when the ON voltage is applied;
a readout circuit (17) configured to convert the electric charges released from the radiation detection elements (7) to actual image data (D) and reads out the image data (D);
a control unit (22), which performs a leak data readout process before radiation imaging, the leak data readout process being a process to convert the electric charges leaking from the radiation detection elements via the switch sections to leak data with the scan driving unit applying the OFF voltage to the scan lines so that the switch sections are in an OFF state, the control unit detecting a start of irradiation when the read-out leak data exceeds a threshold; and
a communication unit (41) with which the image data (D) is transmitted to an external device (60);
a radiation generating device (52) configured to control a radiation source (53) to irradiate the radiation imaging device (1);
a console (60) configured to generate a preview image (p pre) and a radiation image on the basis of the actual image data (D) received from the radiation imaging device (1); and
a portable terminal (70) configured to transmit a completion signal to the console (60) when the portable terminal (70) receives an input of information indicating that positioning of the radiation imaging device (1) has been completed,
wherein a power consumption mode of the radiation imaging device (1) is switchable between a radiographic mode and a sleep mode, the radiographic mode being a mode in which electric power is supplied from a battery (24) to functional parts including at least the scan driving unit (15), the readout circuit (17), and the control unit (22) so that radiation imaging can be performed, and the sleep mode being a mode in which the radiation imaging is not performed with the electric power supplied only to a necessary functional part including at least the communication unit (41);
wherein the radiation imaging device (1) is configured to switch the power consumption mode from the sleep mode to the radiographic mode to start the leak data readout process in response to receipt of a wake-up signal from the console (60) which has received the completion signal from the portable terminal (70); and
wherein, when the control unit (22) of the radiation imaging device (1) detects the start of irradiation, the control unit (22) is configured to change to an electric charge accumulation state and then allows the actual image data (D) to be read out from the radiation detection elements (7) to transmit part of the read-out actual image data (D) to the console (60) as data for preview image, the electric charge accumulation state being a state in which the scan driving unit (15) applies the OFF voltage to the scan lines (5) to accumulate electric charges generated due to the irradiation in the radiation detection elements (7); and
wherein the console (60) is configured to generate a preview image (p pre) on the basis of the data for preview image in response to receipt of the data for preview image from the radiation imaging device (1).

## Patentansprüche

1. Strahlungsbildgebungsystem (100), das Folgendes umfasst:
ein tragbares Strahlungsbildgebungsgerät (1), das Folgendes umfasst:
mehrere Abtastleitungen (5);
mehrere Signalleitungen (6), so angeordnet, dass sie die Abtastleitungen (5) schneiden;
mehrere Strahlungsdetektionselemente (7), die zweidimensional in kleinen Regionen (r) angeordnet sind, die von den Abtastleitungen (5) und den Signalleitungen (6) gebildet werden;
eine Abtasttreibereinheit (15), die eine Einschaltspannung oder eine Ausschaltspannung an die Abtastleitungen (5) anlegt;
mehrere Schaltsektionen (8), die mit den Abtastleitungen (5) verbunden sind und elektrische Ladungen, die sich in den Strahlungsdetektionselementen (7) angesammelt haben, zu den Signalleitungen freisetzen, wenn die Einschaltspannung angelegt wird;
eine Ausleseschaltung (17), die die von den Strahlungsdetektionselementen (7) freigesetzten elektrischen Ladungen in tatsächliche Bilddaten (D) umwandelt und die tatsächlichen Bilddaten (D) ausliest;
eine Steuereinheit (22), die einen Leckdatenausleseprozess vor der Strahlungsbildgebung durchführt, wobei der Leckdatenausleseprozess ein Prozess zum Umwandeln der aus den Strahlungsdetektionselementen leckenden elektrischen Ladungen über die Schaltsektionen in Leckdaten ist, wobei die Abtasttreibereinheit die Ausschaltspannung an die Abtastleitungen anlegt, so dass die Schaltsektionen in einem Ausschaltzustand sind, wobei die Steuereinheit einen Bestrahlungsstart detektiert, wenn die ausgelesenen Leckdaten eine Schwelle übersteigen; und
eine Kommunikationseinheit (41), mit der die Bilddaten (D) zu einem externen Gerät (60) übertragen werden;
ein Strahlungserzeugungsgerät (52), das eine Strahlungsquelle (53) zum Bestrahlen des Strahlungsbildgebungsgeräts (1) steuert;
eine Konsole (60), die ein Voransichtsbild (p pre) und ein Strahlungsbild auf der Basis der von dem Strahlungsbildgebungsgerät (1) empfangenen tatsächlichen Bilddaten (D) erzeugt; und
ein tragbares Endgerät (70), das ein Vollendungssignal zu der Konsole (60) überträgt, wenn das tragbare Endgerät (70) eine Informationseingabe erhält, die anzeigt, dass die Positionierung des Strahlungsbildgebungsgeräts (1) vollendet wurde,
wobei die Steuereinheit (22) des Strahlungsbildgebungsgerätes (1), wenn sie den Bestrahlungsstart detektiert, in einen elektrischen Ladungsakkumulationszustand wechselt und dann das Auslesen der tatsächlichen Bilddaten (D) aus den Strahlungsdetektionselementen (7) zulässt, um einen Teil der ausgelesenen tatsächlichen Bilddaten (D) zu der Konsole (60) als Daten für ein Voransichtsbild zu übertragen, wobei der elektrische Ladungsakkumulationszustand ein Zustand ist, in dem die Abtasttreibereinheit (15) die Ausschaltspannung an die Abtastleitungen (5) anlegt, um aufgrund der Bestrahlung in den Strahlungsdetektionselementen (7) erzeugte elektrische Ladungen zu akkumulieren;
wobei, wenn die Konsole (60) die Daten für das Voransichtsbild von dem Strahlungsbildgebungsgerät (1) vor dem Empfang des Vollendungssignals von dem tragbaren Endgerät (70) empfängt, die Konsole (60) ein Löschsignal zu dem Strahlungsbildgebungsgerät (1) überträgt, einen Prozess stoppt, der von dem Strahlungsbildgebungsgerät (1) durchgeführt wird, und bewirkt, dass die Auslesung der Leckdaten (d) für den Bestrahlungsstartdetektionsprozess erfolgt, bevor die Strahlungsbildgebung wieder aufgenommen wird; und
wobei, wenn die Konsole (60) die Daten für ein Voransichtsbild von dem Strahlungsbildgebungsgerät (1) nach dem Empfang des Vollendungssignals von dem tragbaren Endgerät (70) empfängt, die Konsole (60) das Voransichtsbild (p pre) auf der Basis der Daten für das Voransichtsbild erzeugt.

2. Strahlungsbildgebungssystem (100) nach Anspruch 1,
wobei das Strahlungsbildgebungsgerät (1) einen Versatzdaten-(O)-Ausleseprozess zum Auslesen von Versatzdaten (0) durchführt, die mit einem Versatz äquivalent sind, der von dunklen elektrischen Ladungen resultiert, die über den tatsächlichen Bilddaten (D) überlagert sind, und die Versatzdaten (0) zusammen mit dem Rest der Bilddaten (D) zu der Konsole (60) überträgt, nachdem das Strahlungsbildgebungsgerät (1) die Daten für das Voransichtsbild übertragen hat; und
wobei das Strahlungsbildgebungsgerät (1) das Auslesen und Übertragen der Versatzdaten (0) als Reaktion auf den Empfang des Löschsignals von der Konsole (60) stoppt und den Leckdatenausleseprozess vor der Strahlungsbildgebung oder den Bilddatenausleseprozess zum Auslesen der Bilddaten für eine Bestrahlungsstartdetektion vor der Strahlungsbildgebung wieder aufnimmt.

3. Strahlungsbildgebungssystem nach Anspruch 1 oder 2,
wobei die Konsole ein Signal, das den Empfang des Vollendungssignals repräsentiert, zu dem Strahlungsbildgebungsgerät als Reaktion auf den Empfang des Vollendungssignals von dem tragbaren Endgerät überträgt; und
wobei die Steuereinheit des Strahlungsbildgebungsgeräts, bis zum Empfang des Signals, das den Empfang des Vollendungssignals repräsentiert, die Schwelle auf einem Wert hält, der höher ist als eine normale Schwelle, nachdem die Steuereinheit das den Empfang des Vollendungssignals repräsentierende Signal erhält.

4. Strahlungsbildgebungssystem (100) nach einem der Ansprüche 1 bis 3,
wobei die Konsole (60) das erzeugte Voransichtsbild (p pre) zu dem tragbaren Endgerät (70) überträgt; und
wobei das tragbare Endgerät (70) einen Anzeigeschirm (71) aufweist, auf dem das von der Konsole (60) übertragene Voransichtsbild (p pre) angezeigt wird.

5. Strahlungsbildgebungssystem nach Anspruch 4,
wobei die Konsole nach dem Übertragen des Löschsignals zu dem Strahlungsbildgebungsgerät das Voransichtsbild auf der Basis der Daten für das Voransichtsbild erzeugt und das erzeugte Voransichtsbild zu dem tragbaren Endgerät überträgt.

6. Strahlungsbildgebungssystem nach Anspruch 4 oder Anspruch 5,
wobei die Zulassung oder Nichtzulassung des Voransichtsbildes (p pre) durch das tragbare Endgerät (70) zum Übertragen zur Konsole (60) eingegeben werden kann.

7. Strahlungsbildgebungsystem (100) nach einem der Ansprüche 1 bis 6,
wobei das Strahlungserzeugungsgerät (52) Folgendes umfasst:
einen Expositionsschalter (54), mit dem die Strahlungsquelle (53) zum Beginnen der Bestrahlung angewiesen wird,
eine Falschexpositionsverhütungseinheit (80) zum Verhüten der Betätigung des Expositionsschalters (54), wobei die Falschexpositionsverhütungseinheit (80) öffnen und schließen kann;
wobei die Konsole (60) zum Ändern eines Leistungsaufnahmemodus des Strahlungsbildgebungsgerätes (1) von einem Schlummermodus in einen radiografischen Modus konfiguriert ist, wenn die Konsole (60) detektiert, dass ein Öffnungsvorgang der Falschexpositionsverhütungseinheit (80) durchgeführt wurde und dass der Expositionsschalter (54) in einen operablen Zustand versetzt wurde, wobei der Schlummermodus ein Modus ist, in dem Strahlungsbildgebung nicht mit elektrischer Leistung durchgeführt wird, die von einer Batterie (24) nur zu einem notwendigen Funktionsteil einschließlich wenigstens der Kommunikationseinheit (41) zugeführt wird, und wobei der radiografische Modus ein Modus ist, in dem die elektrische Leistung Funktionsteilen einschließlich wenigstens der Abtasttreibereinheit (15), der Ausleseschaltung (17) und der Steuereinheit (22) zugeführt wird, so dass die Strahlungsbildgebung durchgeführt werden kann; und
wobei, wenn der Leistungsaufnahmemodus in den radiografischen Modus umgeschaltet wird, die Steuereinheit (22) des Strahlungsbildgebungsgerätes (1) den Leckdatenausleseprozess vor dem Strahlungsbildgebungs- oder dem Bilddatenausleseprozess beginnt, um die Bilddaten für die Bestrahlungsstarterkennung vor der Strahlungsbildgebung auszulesen.

8. Strahlungsbildgebungssystem (100), das Folgendes umfasst:
ein tragbares Strahlungsbildgebungsgerät (1), das Folgendes umfasst:
mehrere Abtastleitungen (5);
mehrere Signalleitungen (6), die zum Schneiden der Abtastleitungen angeordnet sind;
mehrere Strahlungsdetektionselemente, die zweidimensional in kleinen Regionen angeordnet sind, die von den Abtastleitungen (5) und den Signalleitungen gebildet werden;
eine Abtasttreibereinheit (15), die Einschaltspannung oder Ausschaltspannung an die Abtastleitungen (5) anlegt;
mehrere Schaltersektionen (8), die mit den Abtastleitungen (5) verbunden sind und in den Strahlungsdetektionselementen (7) akkumulierte elektrische Ladungen zu den Signalleitungen (6) freisetzen, wenn die Einschaltspannung angelegt wird;
eine Ausleseschaltung (17), konfiguriert zum Umwandeln der von den Strahlungsdetektionselementen (7) freigesetzten elektrischen Ladungen in tatsächliche Bilddaten (D) und zum Auslesen der Bilddaten (D);
eine Steuereinheit (22), die einen Leckdatenausleseprozess vor der Strahlungsbildgebung durchführt, wobei der Leckdatenausleseprozess ein Prozess zum Umwandeln der aus den Strahlungsdetektionselementen leckenden elektrischen Ladungen über die Schaltsektionen in Leckdaten ist, wobei die Abtasttreibereinheit die Ausschaltspannung an die Abtastleitungen anlegt, so dass die Schaltsektionen in einem Ausschaltzustand sind, wobei die Steuereinheit einen Bestrahlungsstart detektiert, wenn die ausgelesenen Leckdaten eine Schwelle übersteigen; und
eine Kommunikationseinheit (41), mit der die Bilddaten (D) zu einem externen Gerät (60) übertragen werden;
ein Strahlungserzeugungsgerät (52), konfiguriert zum Steuern einer Strahlungsquelle (53) zum Bestrahlen des Strahlungsbildgebungsgerätes (1);
eine Konsole (60), konfiguriert zum Erzeugen eines Voransichtsbildes (p pre) und eines Strahlungsbildes auf der Basis der von dem Strahlungsbildgebungsgerät (1) empfangenen tatsächlichen Bilddaten (D); und
ein tragbares Endgerät (70), konfiguriert zum Übertragen eines Vollendungssignals zu der Konsole (60), wenn das tragbare Endgerät (70) eine Informationseingabe erhält, die anzeigt, dass die Positionierung des Strahlungsabbildungsgerätes (1) vollendet wurde,
wobei ein Leistungsaufnahmemodus des Strahlungsbildgebungsgerätes (1) zwischen einem radiografischen Modus und einem Schlummermodus umgeschaltet werden kann, wobei der radiografische Modus ein Modus ist, in dem elektrischer Strom von einer Batterie (24) Funktionsteilen einschließlich wenigstens der Abtasttreibereinheit (15), der Ausleseschaltung (17) und der Steuereinheit (22) zugeführt wird, so dass Strahlungsbildgebung durchgeführt werden, und wobei der Schlummermodus ein Modus ist, in dem die Strahlungsbildgebung nicht mit der elektrischen Leistung durchgeführt wird, die nur einem notwendigen Funktionsteil einschließlich wenigstens der Kommunikationseinheit (41) zugeführt wird;
wobei das Strahlungsbildgebungsgerät (1) zum Umschalten des Leistungsaufnahmemodus vom Schlummermodus in den radiografischen Modus konfiguriert ist, um den Leckdatenausleseprozess als Reaktion auf den Empfang eines Aufwecksignals von der Konsole (60) zu starten, die das Vollendungssignal von dem tragbaren Endgerät (70) empfangen hat; und
wobei, wenn die Steuereinheit (22) des Strahlungsbildgebungsgerätes (1) den Bestrahlungsstart detektiert, die Steuereinheit (22) zum Wechseln in einen elektrischen Ladungsakkumulationszustand konfiguriert ist und es dann zulässt, dass die tatsächlichen Bilddaten (D) von den Strahlungsdetektionselementen (7) ausgelesen werden, um einen Teil der ausgelesenen tatsächlichen Bilddaten (D) zu der Konsole (60) als Daten für ein Voransichtsbild zu übertragen, wobei der elektrische Ladungsakkumulationszustand ein Zustand ist, in dem die Abtasttreibereinheit (15) die Ausschaltspannung an die Abtastleitungen (5) anliegt, um aufgrund der Bestrahlung in den Strahlungsdetektionselementen (7) erzeugte elektrische Ladungen zu akkumulieren; und
wobei die Konsole (60) zum Erzeugen eines Voransichtsbildes (p pre) auf der Basis der Daten für das Voransichtsbild als Reaktion auf den Empfang der Daten für das Voransichtsbild von dem Strahlungsbildgebungsgerät (1) konfiguriert ist.

## Revendications

1. Système d'imagerie par rayonnement (100) comprenant :
un dispositif d'imagerie par rayonnement (1) portatif comportant :
une pluralité de lignes de balayage (5) ;
une pluralité de lignes de signaux (6) disposées pour intersecter les lignes de balayage (5) ;
une pluralité d'éléments de détection de rayonnement (7) disposés bidimensionnellement dans de petites régions (r) formées par les lignes de balayage (5) et les lignes de signaux (6) ;
une unité d'excitation de balayage (15) qui applique une tension d'activation ou une tension de désactivation aux lignes de balayage (5) ;
une pluralité de sections de commutation (8) qui sont connectées aux lignes de balayage (5) et qui libèrent des charges électriques accumulées dans les éléments de détection de rayonnement (7) vers les lignes de signaux quand la tension d'activation est appliquée ;
un circuit de lecture (17) qui convertit les charges électriques libérées par les éléments de détection de rayonnement (7) en données d'image effectives (D) et lit les données d'image effectives (D) ;
une unité de commande (22) qui exécute un processus de lecture de données de fuite avant l'imagerie par rayonnement, le processus de lecture de données de fuite étant un processus servant à convertir les charges électriques s'échappant des éléments de détection de rayonnement par l'intermédiaire des sections de commutation en données de fuite avec application de la tension de désactivation aux lignes de balayage par l'unité d'excitation de balayage de telle sorte que les sections de commutation soient dans un état désactivé, l'unité de commande détectant un début d'irradiation quand les données de fuite lues dépassent un seuil ; et
une unité de communication (41) avec laquelle les données d'image (D) sont transmises à un dispositif externe (60) ;
un dispositif de génération de rayonnement (52) qui commande une source de rayonnement (53) pour irradier le dispositif d'imagerie par rayonnement (1) ;
une console (60) qui génère une image de prévisualisation (p pre) et une image de rayonnement en fonction des données d'image effectives (D) reçues depuis le dispositif d'imagerie par rayonnement (1) ; et
un terminal portatif (70) qui transmet un signal d'achèvement à la console (60) quand le terminal portatif (70) reçoit une entrée d'informations indiquant que le positionnement du dispositif d'imagerie par rayonnement (1) est achevé,
dans lequel, quand l'unité de commande (22) du dispositif d'imagerie par rayonnement (1) détecte le début de l'irradiation, l'unité de commande (22) passe à un état d'accumulation de charges électriques puis autorise la lecture des données d'image effectives (D) des éléments de détection de rayonnement (7) pour transmettre une partie des données d'image effectives (D) lues à la console (60) en tant que données d'image de prévisualisation, l'état d'accumulation de charges électriques étant un état dans lequel l'unité d'excitation de balayage (15) applique la tension de désactivation aux lignes de balayage (5) pour accumuler des charges électriques générées en raison de l'irradiation dans les éléments de détection de rayonnement (7) ;
dans lequel, quand la console (60) reçoit les données d'image de prévisualisation depuis le dispositif d'imagerie par rayonnement (1) avant la réception du signal d'achèvement depuis le terminal portatif (70), la console (60) transmet un signal d'annulation au dispositif d'imagerie par rayonnement (1), arrête un processus en cours d'exécution par le dispositif d'imagerie par rayonnement (1), et reprend la lecture des données de fuite (d) du processus de détection de début d'irradiation avant que l'imagerie par rayonnement reprenne ; et
dans lequel, quand la console (60) reçoit les données d'image de prévisualisation depuis le dispositif d'imagerie par rayonnement (1) après la réception du signal d'achèvement depuis le terminal portatif (70), la console (60) génère l'image de prévisualisation (p pre) en fonction des données d'image de prévisualisation.

2. Système d'imagerie par rayonnement (100) selon la revendication 1,
dans lequel le dispositif d'imagerie par rayonnement (1) exécute un processus de lecture de données de décalage (0) pour lire des données de décalage (0) correspondant à un décalage résultant de charges électriques sombres superposées sur les données d'image effectives (D) et transmet les données de décalage (0) avec le reste des données d'image (D) à la console (60) après que le dispositif d'imagerie par rayonnement (1) transmet les données d'image de prévisualisation ; et
dans lequel le dispositif d'imagerie par rayonnement (1) arrête de lire et de transmettre les données de décalage (0) en réponse à la réception du signal d'annulation provenant de la console (60) et reprendre le processus de lecture de données de fuite avant l'imagerie par rayonnement ou le processus de lecture de données d'image pour lire les données d'image de détection de début d'irradiation avant l'imagerie par rayonnement.

3. Système d'imagerie par rayonnement selon l'une quelconque des revendications 1 ou 2,
dans lequel la console transmet un signal représentant la réception du signal d'achèvement au dispositif d'imagerie par rayonnement en réponse à la réception du signal d'achèvement depuis le terminal portatif ; et
dans lequel, jusqu'à ce qu'elle reçoive le signal représentant la réception du signal d'achèvement, l'unité de commande du dispositif d'imagerie par rayonnement maintient le seuil à une valeur supérieure à un seuil normal après que l'unité de commande reçoit le signal représentant la réception du signal d'achèvement.

4. Système d'imagerie par rayonnement (100) selon l'une quelconque des revendications 1 à 3,
dans lequel la console (60) transmet l'image de prévisualisation (p pre) générée au terminal portatif (70) ; et
dans lequel le terminal portatif (70) comporte un écran d'affichage (71) sur lequel l'image de prévisualisation (p pre) transmise par la console (60) est affichée.

5. Système d'imagerie par rayonnement selon la revendication 4,
dans lequel, à la transmission du signal d'annulation au dispositif d'imagerie par rayonnement, la console génère l'image de prévisualisation en fonction des données d'image de prévisualisation et transmet l'image de prévisualisation générée au terminal portatif.

6. Système d'imagerie par rayonnement selon la revendication 4 ou la revendication 5,
dans lequel l'acceptation ou le refus de l'image de prévisualisation (p pre) peut être saisi par le biais du terminal portatif (70) en vue de sa transmission à la console (60).

7. Système d'imagerie par rayonnement (100) selon l'une quelconque des revendications 1 à 6,
dans lequel le dispositif de génération de rayonnement (52) comporte :
un commutateur d'exposition (54) au moyen duquel il est donné instruction à la source de rayonnement (53) de commencer l'irradiation, et
une unité d'empêchement de mauvaise exposition (80) pour empêcher l'actionnement du commutateur d'exposition (54), l'unité d'empêchement de mauvaise exposition (80) étant capable d'ouverture et de fermeture ;
dans lequel la console (60) est configurée pour changer un mode de consommation de puissance du dispositif d'imagerie par rayonnement (1) d'un mode de veille à un mode radiographique quand la console (60) détecte qu'une opération d'ouverture de l'unité d'empêchement de mauvaise exposition (80) a été effectuée et que le commutateur d'exposition (54) a été placé en un état d'actionnement, le mode de veille étant un mode dans lequel l'imagerie par rayonnement n'est pas effectuée, la puissance électrique étant fournie par une batterie (24) uniquement à une partie fonctionnelle nécessaire comportant au moins l'unité de communication (41), et le mode radiographique étant un mode dans lequel la puissance électrique est fournie à des parties fonctionnelles comportant au moins l'unité d'excitation de balayage (15), le circuit de lecture (17), et l'unité de commande (22) de telle sorte que l'imagerie par rayonnement puisse être effectuée ; et
dans lequel, quand le mode de consommation de puissance est commuté sur le mode radiographique, l'unité de commande (22) du dispositif d'imagerie par rayonnement (1) commence le processus de lecture de données de fuite avant l'imagerie par rayonnement ou le processus de lecture de données d'image pour lire les données d'image de détection de début d'irradiation avant l'imagerie par rayonnement.

8. Système d'imagerie par rayonnement (100) comprenant :
un dispositif d'imagerie par rayonnement portatif (1) comportant :
une pluralité de lignes de balayage (5) ;
une pluralité de lignes de signaux (6) disposées pour intersecter les lignes de balayage ;
une pluralité d'éléments de détection de rayonnement disposés bidimensionnellement dans de petites régions formées par les lignes de balayage (5) et les lignes de signaux ;
une unité d'excitation de balayage (15) qui applique une tension d'activation ou une tension de désactivation aux lignes de balayage (5) ;
une pluralité de sections de commutation (8) qui sont connectées aux lignes de balayage (5) et qui libèrent les charges électriques accumulées dans les éléments de détection de rayonnement (7) vers les lignes de signaux (6) quand la tension d'activation est appliquée ;
un circuit de lecture (17) configuré pour convertir les charges électriques libérées par les éléments de détection de rayonnement (7) en données d'image effectives (D) et lit les données d'image (D) ;
une unité de commande (22) qui exécute un processus de lecture de données de fuite avant l'imagerie par rayonnement, le processus de lecture de données de fuite étant un processus servant à convertir les charges électriques s'échappant des éléments de détection de rayonnement par l'intermédiaire des sections de commutation en données de fuite avec application de la tension de désactivation aux lignes de balayage par l'unité d'excitation de balayage de telle sorte que les sections de commutation soient dans un état désactivé, l'unité de commande détectant un début d'irradiation quand les données de fuite lues dépassent un seuil ; et
une unité de communication (41) avec laquelle les données d'image (D) sont transmises à un dispositif externe (60) ;
un dispositif de génération de rayonnement (52) configuré pour commander une source de rayonnement (53) pour irradier le dispositif d'imagerie par rayonnement (1) ;
une console (60) configurée pour générer une image de prévisualisation (p pre) et une image de rayonnement en fonction des données d'image effectives (D) reçues du dispositif d'imagerie par rayonnement (1) ; et
un terminal portatif (70) configuré pour transmettre un signal d'achèvement à la console (60) quand le terminal portatif (70) reçoit une entrée d'informations indiquant que le positionnement du dispositif d'imagerie par rayonnement (1) est achevé,
dans lequel un mode de consommation de puissance du dispositif d'imagerie par rayonnement (1) peut être commuté entre un mode radiographique et un mode de veille, le mode radiographique étant un mode dans lequel une puissance électrique est fournie par une batterie (24) à des parties fonctionnelles comportant au moins l'unité d'excitation de balayage (15), le circuit de lecture (17), et l'unité de commande (22) de telle sorte que l'imagerie par rayonnement puisse être effectuée, et le mode de veille étant un mode dans lequel l'imagerie par rayonnement n'est pas effectuée, la puissance électrique étant fournie uniquement à une partie fonctionnelle nécessaire comportant au moins l'unité de communication (41) ;
dans lequel le dispositif d'imagerie par rayonnement (1) est configuré pour commuter le mode de consommation de puissance du mode de veille sur le mode radiographique pour lancer le processus de lecture de données de fuite en réponse à la réception d'un signal de réveil provenant de la console (60) qui a reçu le signal d'achèvement depuis le terminal portatif (70) ; et
dans lequel, quand l'unité de commande (22) du dispositif d'imagerie par rayonnement (1) détecte le début de l'irradiation, l'unité de commande (22) est configurée pour passer à un état d'accumulation de charges électriques puis autorise la lecture des données d'image effectives (D) des éléments de détection de rayonnement (7) pour transmettre une partie des données d'image effectives (D) lues à la console (60) en tant que données d'image de prévisualisation, l'état d'accumulation de charges électriques étant un état dans lequel l'unité d'excitation de balayage (15) applique la tension de désactivation aux lignes de balayage (5) pour accumuler les charges électriques générées en raison de l'irradiation dans les éléments de détection de rayonnement (7) ; et
dans lequel la console (60) est configurée pour générer une image de prévisualisation (p pre) en fonction des données d'image de prévisualisation en réponse à la réception des données d'image de prévisualisation depuis le dispositif d'imagerie par rayonnement (1).
